# EUROPEAN PATENT SPECIFICATION

(11) **EP 1 240 823 B1**
(45) Date of publication and mention of the grant of the patent: **20.08.2008**
(21) Application number: 00970097.2
(22) Date of filing: 26.10.2000
(51) Int. Cl.: A01K 67/027, G01N 33/50, G01N 33/15, A61K 45/00, A61P 9/12, A61P 3/10, A61P 25/04, C12N 15/12, C07K 14/705

(54) **N-CALCIUM CHANNEL KNOCKOUT ANIMAL**
N-KALZIUM-KANAL-"KNOCKOUT-TIER"
ANIMAL KNOCKOUT A CANAL N-CALCIUM

(30) Priority: 26.10.1999 JP 30380999; 16.02.2000 JP 2000037839; 31.08.2000 JP 2000261979
(43) Date of publication of application: 18.09.2002
(73) Proprietor: Eisai R&D Management Co., Ltd., Tokyo 112-8088 (JP)
(72) Inventor: INO, Mitsuhiro, Ushiku-shi, Ibaraki 300-1234 (JP); MIYAMOTO, Norimasa, Tsukuba-shi, Ibaraki 305-0051 (JP); TAKAHASHI, Eiki, Ushiku-shi, Ibaraki 300-1237 (JP); OKI, Toru, Ushiku-shi, Ibaraki 300-1222 (JP); YOSHINAGA, Takashi, Tsukuba-shi, Ibaraki 305-0042 (JP); HATAKEYAMA, Shinji, Ushiku-shi, Ibaraki 300-1234 (JP); NIIDOME, Tetsuhiro, Ryugasaki-shi, Ibaraki 301-0043 (JP); SAWADA, Kohei, Kitasoma-gun, Ibaraki 302-0102 (JP); NISHIZAWA, Yukio, Tsukuba-shi, Ibaraki 305-0035 (JP); TANAKA, Isao, Tsukuba-shi, Ibaraki 305-0051 (JP)
(74) Representative: Dean, John Paul
(86) International application number: PCT/JP2000/007503
(87) International publication number: WO 2001/030137

(56) References cited:
- WO-A-99/46383
- WO-A1-93/04083
- US-A1- 5 677 288
- FEBS LETTERS vol. 338, 1994, pages 1 - 5, XP002936371
- TRENDS IN PHARMACOLOGICAL SCIENCES vol. 18, no. 10, 1997, pages 363 - 371, XP002936372
- PROC. NATL. ACAD. SCI. USA vol. 95, 1998, pages 12010 - 12015, XP002936373
- PROC. NATL. ACAD. SCI. USA vol. 97, 23 March 2000, pages 6132 - 6137, XP002936374
- PROC. NATL. ACAD. SCI. USA vol. 96, 21 December 1999, pages 15245 - 15250, XP002936375
- SCIENCE vol. 257, 1992, pages 389 - 395, XP002936376
- NATURE vol. 336, 1988, pages 348 - 352, XP002936377
- CLINICAL ENGINEERING vol. 7, no. 12, 1996, pages 1085 - 1090, XP002936378
- KYUSHU SHIKA GAKKAI ZASSHI vol. 54, no. 1, 25 February 2000, pages 162 - 171, XP002936379
- JOURNAL OF CARDIOVASCULAR PHARMACOLOGY vol. 16, no. 4, 1990, pages 675 - 680, XP002936380
- JOURNAL OF PHARMACOLOGY AND EXPERIMENTAL THERAPEUTICS vol. 278, no. 3, 1996, pages 1392 - 1407, XP002936381

## Description

### Technical Field

The present invention relates to an animal deficient in N-type calcium channel and use thereof.

### Background Art

Calcium channels (Ca channels) are membrane proteins that transmit information into cells by controlling influx of Ca²⁺ into the cells. In particular, voltage-dependent Ca channels present in excitatory cells such as nerve cells and muscle cells are proteins that play an important role of converting information transmitted through changes in membrane potential, into intracellular information which is an increase in Ca²⁺ concentration.

Various voltage-dependent Ca channels have been identified from nerve cells and muscle cells (Bean, B.P. et al, Ann. Rev. Physiol., 51, pp.367-384, 1989; Hess P., Ann. Rev. Neurosci., 56, p.337, 1990), and these are classified into six types (L, N, P, Q, R and T) according to their electrophysiological properties and susceptibility to antagonists.

Among these Ca channels, N-type Ca channel is a Ca channel characterized in that Ca²⁺ influx is inhibited by a peptide toxin isolated from cone shell, ω-conotoxin GVIA.

Calcium antagonists are widely used as antianginal drugs, antiarrhythmic drugs and therapeutic agents for hypertension, and their action mechanism is based on relaxation of vascular smooth muscles or suppression of myocardial contraction by inhibition of the Ca²⁺ influx into a cell through a specific binding to the L-type Ca channel present in a cell membrane. Meanwhile, it is being revealed that Ca²⁺ is an important factor for normal functions in nerves, such as release of nerve transmitter substances, formation of impulse patterns and outgrowth of neurites, while a Ca²⁺ kinetics change is deeply involved in diseases such as delayed nerve cell death after cerebral ischemia and a certain kind of epilepsy (Siesjo, Mayo Clin Proc., 61, p.299, 1986). Over the last few years, existence of P-, N-, Q- and R-type Ca channels, which are specifically present in nerves, were confirmed in addition to L-type and T-type. Roles of these Ca channels in nervous functions draw attentions, and novel calcium antagonists targeting them are being actively developed at the same time.

In particular, it has been reported that the N-type Ca channel is expressed at nerve endings of the autonomic nervous system, and its role in control through autonomic nerves is attracting attentions (Lane D.H. et al., Science, 239, pp.57-61, 1988; Diane L, et al., Nature, 340, pp.639-642, 1989).

Functions of the N-type Ca channel have hitherto been evaluated by conducting 1) an *in vitro* experiment using synaptosomes or cultured nerve cells or 2) an *in vivo* experiment using administration of ω-conotoxin GVIA. Since 1) is an *in vitro* experiment, it is not suitable for precise evaluation of the N-type Ca channel functions in living bodies. On the other hand, although 2) is an *in vivo* experiment, this is not suitable for precise evaluation of the N-type Ca channel functions in living bodies either because (1) selectivity of w-conotoxin GVIA has not been completely elucidated, (2) ω-conotoxin GVIA is a peptide and hence it does not have sufficient permeability to a nerve cell, (3) a chronic-stage experiment using administration of ω-conotoxin GVIA is difficult and so forth.

### Disclosure of the Invention

In order to overcome the aforementioned drawbacks, preparation of an N-type Ca channel knockout mouse that is deficient only in the N-type Ca channel and can be used for a chronic-stage experiment has been strongly desired.

Accordingly, an object of the present invention is to prepare a knockout mouse which lacks α_{1B} subunit of the N-type Ca channel (referred to as "N-KO mouse" hereinafter). By using such a mouse, what functions the N-type Ca channel is actually responsible for in living bodies can be elucidated, which N-type Ca channel is considered to be expressed at nerve terminals of the central nervous system and the peripheral nervous system and plays an important role in maintenance of homeostasis of living bodies.

The N-KO mouse may not be able to maintain homeostasis through the autonomic nervous system, especially it cannot control blood pressure, and hence it may not survive normally. However, it was considered that, even though the N-KO mouse could not survive normally, the N-type Ca channel functions could be deduced from abnormalities observed in the N-KO mouse. Thus, it was attempted to prepare an N-KO mouse in which a gene coding for the α_{1B} subunit of the N-type Ca channel was disrupted by targeted disruption.

As a result, it was revealed that the N-KO mouse could undergo ontogenesis and growth and could produce offspring. Moreover, it was electrophysiologically proved that Ca²⁺ influx that is inhibited by ω-conotoxin GVIA was not observed in nerve cells in dorsal root ganglia prepared from the N-KO mouse, and hence it was confirmed that the N-KO mouse lacked functional N-type Ca channel.

As a result of further studies, it was also revealed that the N-KO mouse had characteristics unique to deficiency in N-type Ca channel such as no blood-pressure reflex through nervous systems, insensitity to pain and low blood sugar level compared with a wild-type mouse, and that the N-KO mouse was useful for analysis of N-type Ca channel functions in living bodies. Thus, the present invention has been accomplished.

That is, the present invention provides a non-human animal in which a gene coding for an N-type Ca channel is disrupted to lack functional N-type Ca channel (hereinafter, also referred to as "animal of the present invention"). The non-human animal is a mouse.

The gene coding for the N-type Ca channel is a gene coding for an α_{1B} subunit of the N-type Ca channel. More specifically, there can be mentioned a gene comprising DNA defined in the following (a) or (b):
(a) DNA which comprises the nucleotide sequence of SEQ ID NO: 1;
(b) DNA which is hybridizable with DNA comprising the nucleotide sequence of SEQ ID NO: 1 under a stringent condition and codes for an α_{1B} subunit of functional N-type calcium channel.

The present invention also provides a method for determining an action of a substance, which comprises steps of administering a substance to the animal of the present invention and determining an action of the substance on the animal (hereafter, also referred to as "the determination method of the present invention").

The determination method of the present invention preferably comprises steps of administering a substance to the animal of the present invention and a wild-type animal, and comparing actions of the substance on the animal of the present invention and the wild-type animal to determine the action of the substance on the N-type calcium channel.

The present invention further provides a method for screening for a substance having a pharmacological action, which comprises a step of determining a pharmacological action of a substance by the determination method of the present invention.

As the pharmacological action, there can be mentioned an action for lowering blood pressure, an analgesic action and an action for lowering blood sugar level. Substances having such pharmacological actions can be used to manufacture hypotensive drugs, analgesic drugs and hypoglycemic drugs comprising these substances as active ingredients, respectively.

### Brief Description of the Drawings

Fig. 1 shows a restriction enzyme map of a phage DNA clone and pBS59/63/58n.
Fig. 2 shows preparation of a targeting vector.
Fig. 3 shows preparation of a targeting vector.
Fig. 4 shows preparation of a targeting vector.
Fig. 5 shows comparison of electric currents passed through N-type Ca channels of an N-KO mouse and a wild-type mouse.
Fig. 6 shows comparison of heart rate and blood pressure of an N-KO mouse and those of a wild-type mouse.
Fig. 7 shows comparison of changes in blood pressure of an N-KO mouse and a wild-type mouse, to ω-conotoxin was administered.
Fig. 8 shows comparison of changes in blood pressure of an N-KO mouse and a wild-type mouse, which were subjected to bilateral carotid occlusion (BCO).
Fig. 9 shows comparison of susceptibilities of an N-KO mouse and a wild-type mouse to pain in a formalin test.
Fig. 10 shows comparison of blood sugar levels of an N-KO mouse and a wild-type mouse.
Fig. 11 shows comparison of blood sugar levels of an N-KO mouse and a wild-type mouse after glucose administration.
Fig. 12 shows comparison of blood insulin level of an N-KO mouse and a wild-type mouse after glucose administration.
Fig. 13 shows comparison of autonomic innervation for atrial cardiac muscle contractile forces in an N-KO mouse and a wild-type mouse.

### Best Mode for Carrying out the Invention

Hereafter, embodiments of the present invention will be explained in detail.

As described above, the inventors of the present invention found that a mouse deficient in functional N-type Ca channel underwent ontogenesis and growth and could produce offspring, and that this mouse was useful for analysis of N-type Ca channel functions in living bodies. The animal of the present invention is based on these findings and is characterized by being a non-human animal wherein a gene coding for the N-type Ca channel is disrupted to lack functional N-type Ca channel.

Disruption of a gene means introducing a mutation into the gene so that function of its gene product is lost. As a method for disrupting a gene, there can be mentioned targeted disruption. The targeted disruption is a method for disrupting a gene by gene targeting, and refers to a mutation introducing technique wherein DNA having a nucleotide sequence of a target gene into which a mutation by which function of the gene product is lost is introduced, preferably DNA having a nucleotide sequence of a target gene into which a selective marker, more preferably a drug resistance gene is inserted, so that function of the gene product is lost, is introduced into a cell, and a cell having undergone homologous recombination between the introduced DNA and the target gene is selected (Suzanne L. et al., Nature, 336, p.348, 1988). The targeted disruption mentioned herein is an example of a technique for disrupting the gene coding for an N-type Ca channel based on information about the nucleotide sequence of the gene, and any techniques fall within the scope of the present invention so long as a gene is disrupted based on information about the nucleotide sequence thereof.

Further, lack of a functional N-type Ca channel means that there is no longer substantial influx of Ca²⁺ passed through the N-type Ca channel and can be verified by absence of substantial influx of Ca²⁺ inhibited by ω-conotoxin GVIA. The ω-conotoxin GVIA referred to herein is a peptide purified from cone shell (*Conus geographus*) toxin (Baldomero, M.O. et al., Biochemistry, 23, p.5087, 1984), and it is characterized by the amino acid sequence of SEQ ID NO: 3.

A gene coding for an N-type Ca channel means a gene coding for a constitutional subunit contained only in the N-type Ca channel, for example, the α_{1B} subunit.

Specific examples of the gene coding for the α_{1B} subunit include a gene having DNA defined in the following (a) or (b):
(a) DNA which comprises the nucleotide sequence of SEQ ID NO: 1;
(b) DNA which is hybridizable with DNA comprising the nucleotide sequence of SEQ ID NO: 1 under a stringent condition and codes for the α_{1B} subunit of a functional N-type calcium channel.

An example of the stringent condition mentioned herein include the conditions of hybridization at 65°C in 4 x SSC and subsequent washing at 65°C in 0.1 x SSC for 1 hour. The stringent condition may alternatively be 42°C, 4 x SSC in 50% formamide.

The non-human animal is preferably a rodent, more preferably a mouse.

The animal of the present invention can be prepared according to a usual method for preparing a knockout animal by gene targeting except that the gene coding for an N-type Ca channel is used as a target gene.

Hereafter, cloning of the N-type Ca channel α_{1B} subunit gene, construction of a targeting vector used in targeted disruption and acquisition of an embryonic stem cell (ES cell) having undergone homologous recombination will be explained in this order by exemplifying targeted disruption of a gene coding for an N-type Ca channel.

### 1. Cloning of DNA including part of N-type Ca channel α_{1B} subunit gene

DNA coding for the N-type Ca channel α_{1B} subunit can be obtained by designing primers based on the nucleotide sequence described in Thlerry, C. et al., FEBS Letters, 338, p.1, 1994 and performing PCR using non-human animal genomic DNA or cDNA or performing RT-PCR using non-human animal RNA. Alternatively, a probe may be synthesized based on the nucleotide sequence described in the aforementioned reference, and clones hybridizable with the probe may be selected from a non-human animal genomic DNA library or cDNA library and determined for the nucleotide sequences to select a clone containing the N-type Ca channel α_{1B} subunit gene or a part thereof comprising a nucleotide sequence of preferably 500 bp or more, more preferably 1 kbp or more.

A restriction enzyme map is prepared by determining restriction enzyme sites contained in the cloned DNA. In the case where a clone containing DNA of a length enough to cause homologous recombination, i.e., a clone of preferably 7 kbp or longer, more preferably 10 kbp or longer, is not obtained, DNAs may be excised from a plurality of clones at appropriate restriction enzyme sites and ligated.

### 2. Construction of targeting vector

A positive selection marker such as a drug resistance gene, preferably a neomycin resistance gene, is introduced into a restriction enzyme site of an exon region in the obtained DNA having a length enough to cause homologous recombination. Further, a part of the exon may be eliminated and replaced with a drug resistance gene. When there is no appropriate restriction enzyme site, appropriate restriction enzyme sites may be introduced by PCR using a primer designed so as to include restriction enzyme sites, ligation of oligonucleotides including restriction enzyme sites and so forth.

Preferably, the vector includes a negative selection marker such as thymidine kinase gene and diphtheria toxin gene in order to eliminate ES cells that do not undergo homologous recombination between the introduced DNA and the N-type Ca channel α_{1B} subunit gene in which the introduced DNA is inserted into a site that is not the N-type Ca channel α_{1B} subunit gene.

These recombinant DNA techniques for manipulating DNA nucleotide sequences can be implemented according to, for example, the methods described in Sambruck, J., Fritsch, E.F., and Maniatis, T., "Molecular Cloning: A Laboratory Manual", Cold Spring Harbor Laboratory, Cold Spring Harbor, New York, 1989, but such techniques are not limited to these methods so long as appropriate recombinant DNA can be obtained.

### 3. Acquisition of embryonic stem cell (ES cell) having undergone homologous recombination

The prepared targeting vector is digested with restriction enzymes to form linear DNA, purified by, for example, phenol/chloroform extraction, agarose electrophoresis, ultracentrifugation and so forth and transfected into an ES cell, for example, TT2. Examples of the transfection method include electroporation, lipofection and so forth, but the present invention is not limited to these methods.

The transfected cell is cultured in an appropriate selection medium, for example, a selection medium containing neomycin and ganciclovir when a targeting vector incorporated with a neomycin resistance gene and a thymidine kinase gene is constructed.

It is readily confirmed by PCR or the like that an introduced gene, for example, a neomycin resistance gene, is incorporated into an ES cell that shows resistance to the both drugs and grows. Further, occurrence of the homologous recombination can also be confirmed by Southern blotting analysis using a 5' upstream or 3' downstream part of DNA outside the targeting vector as a probe. Further, it can be confirmed by Southern blotting analysis using DNA with the targeting vector as a probe that the targeting vector is not randomly inserted. An ES cell having undergone homologous recombination can be obtained by combining these methods.

An example of a method for preparing a knockout mouse will be described below, but the present invention is not limited to this example.

A knockout mouse is prepared by taking steps of collection of an 8-cell embryo or a blastocyst after fertilization, microinjection of an ES cell having undergone homologous recombination, implantation of a manipulated egg into a pseudopregnant mouse, delivery from the pseudopregnant mouse and raising of offspring, selection of a transgenic mouse by PCR and Southern blotting, and establishment of pedigree of mice having the introduced gene (Yagi, T. et al., Analytical Biochem., 214, p.70, 1993).

### 1. Collection of 8-cell embryo or blastocyst

As for fertilized eggs, 5 IU of pregnant mare's serum gonadotropin and 2.5 IU of human chorionic gonadotropin are intraperitoneally administered to a female mouse in order to induce superovulation, and an 8-cell embryo is obtained from the female mouse on day 2.5 after fertilization by the oviduct-uterus perfusion method. When a blastocyst is used, the uterus of a female mouse is removed on day 3.5 after fertilization and an embryo is obtained by uterus perfusion.

### 2. Microinjection of ES cell having undergone homologous recombination

An ES cell having undergone homologous recombination is microinjected into the obtained 8-cell embryo or blastocyst. The microinjection can be performed under an inverted microscope by using a micromanipulator, microinjector, injection pipette and holding pipette based on, for example, the descriptions in Hogan, B.L.M., "A laboratory Manual", Cold Spring Harbor Laboratory Press, Cold Spring Harbor, New York, 1986 (Yagi, T. et al., Analytical Biochem., 214, p.70, 1993). Further, as an injection dish, for example, there are used 5-µl medium droplets and droplets containing floating ES cells formed on Falcon 3002 (Becton Dickinson Labware), on which liquid paraffin is overlaid. Hereinafter, an 8-cell embryo or blastocyst microinjected with an ES cell having undergone homologous recombination is referred to as a manipulated egg.

### 3. Implantation of manipulated egg into pseudopregnant mouse

A vasoligated male mouse and a normal female mouse are mated to prepare a pseudopregnant mouse, into which a manipulated egg is implanted. Implantation of a manipulated egg can be performed based on, for example, the descriptions in Hogan, B.L.M., "A laboratory Manual", Cold Spring Harbor Laboratory Press, Cold Spring Harbor, New York, 1986 and Yagi, T. et al., Analytical Biochem., 214, P.70, 1993. An example of specific procedure will be described below, but the present invention is not limited to this example.

A pseudopregnant mouse is generally anesthetized by using, for example, 50 mg/kg body weight of pentobarbital sodium. Then, both flanks are incised about 1 cm to expose the ovary and the oviduct. The bursa ovarica is incised by using tweezers under a stereoscopic microscope to expose the fimbriae tubae. Subsequently, 7 to 8 manipulated eggs per oviduct are introduced into the fimbriae tubae. At this time, implantation of the manipulated eggs into the oviduct is confirmed by micro air bubbles inserted together with the manipulated eggs. Then, the oviduct and the ovary are returned to the abdominal cavity, both of the incision sites are sutured, and the mouse is awakened from the anesthesia. In some cases, manipulated eggs may be cultured until the following day to be developed into a blastocyst and then implanted into the uterus.

### 4. Delivery from pseudopregnant mouse and raising of offspring

In many cases, offspring mice can be obtained on day 17 after the implantation. The offspring mice are usually chimeric mice obtained from the ES cell having undergone homologous recombination and a cell of the mouse from which the fertilized egg is collected. For example, when TT2 is used as an ES cell and injected into an 8-cell embryo collected from ICR, an offspring mouse having a high chimeric rate shows an agouti-dominant coat color, while a mouse having a low chimeric rate shows a white-dominant coat color.

### 5. Screening for gene-introduced mouse by PCR and Southern blotting

Whether the gene is present in a germ cell can be readily confirmed by the coat color of an offspring mouse obtained by mating a mouse of interest with a mouse having a white coat color, for example, ICR. Alternatively, since a mouse having a high chimeric rate is expected to also have a germ cell containing the introduced gene, the presence or absence of the gene can be confirmed by using a mouse having a chimeric rate as high as possible for mating, extracting DNA from the tail of the obtained offspring mouse and subjecting its DNA to PCR. Further, a genotype can be more reliably identified by performing Southern blotting analysis instead of PCR.

### 6. Establishment of lineage of mice having introduced gene

An N-KO mouse in which the introduced gene homozygously exists can be obtained among the offspring mice obtained by mating heterozygous mice (hereinafter, referred to as He mice) with each other. The N-KO mouse can be obtained by mating He mice with each other, a He mouse with an N-KO mouse, or N-KO mice with each other.

The presence or absence of expression of the α_{1B} subunit mRNA in the N-KO mouse can be confirmed by Northern blotting analysis, RT-PCR, RNase protection assay, *in situ* hybridization or the like. Further, expression of the α_{1B} subunit protein can be confirmed by immunohistochemical staining, labeled ω-conotoxin or the like. Further, a function of an N-type Ca channel including the α_{1B} subunit can also be confirmed by an electrophysiological method or the like.

Moreover, as described above, the inventors of the present invention found that an animal lacking the gene coding for an N-type Ca channel lost blood pressure control through the autonomic nervous system, had defects in a mechanism for transmitting pain, especially second phase pain that appears in a delayed manner, and had abnormality in blood sugar level control. That is, they found that the animal had unique characteristics associated with the deletion of the gene coding for an N-type Ca channel. The determination method of the present invention is based on these findings and it is a method for determining an action of a substance that comprises steps of administering a substance such as a compound to the animal of the present invention and determining the action of the substance on the animal.

The determination method of the present invention preferably comprises steps of administering a substance to the animal of the present invention and a wild-type animal and comparing actions of the substance on the animal of the present invention and the wild-type animal to determine the action of the substance on the N-type Ca channel. The influence of the substance on the N-type Ca channel can be examined by determining the action on the N-type Ca channel.

An action refers to an action on a characteristic unique to the animal. For example, when attention is paid to abnormality of the animal in blood pressure control, transmission of pain or blood sugar level control, the action refers to an action on the blood pressure, pain or blood sugar level. However, the action is not limited to these examples so long as the action is associated with the characteristics unique to the animal. These actions can be determined as activities of the substances.

Further, a wild type means that functional N-type Ca channel is not lost.

The present invention further provides a method for screening for a substance having a pharmacological action by using the animal of the present invention (non-human animal deficient in N-type Ca channel). Specifically, a method for screening for a substance having a pharmacological action, for example, a substance acting on blood pressure, transmission of pain or blood sugar level of the animal (that is, a substance having an action for lowering blood pressure, a substance having an analgesic action or a substance having an action for lowering blood sugar level) by using the determination method of the present invention.

As examples, a substance having an action for lowering blood pressure, a substance having an analgesic action or a substance having an action for lowering blood sugar level will be described below in this order. However, any substances fall within the scope of the present invention so long as they are obtained by utilizing a screening system using the animal of the present invention.

### 1. Method for screening for substance having action for lowering blood pressure (hypotensive drug)

Candidate substances can be screened for a substance having an action for lowering blood pressure through blocking the influx of Ca²⁺ passed through N-type Ca channel by administering each of the candidate substances to a non-human animal deficient in the N-type Ca channel (N-KO animal) and a wild-type animal not deficient in the channel (Wt animal) and selecting a drug that lowers blood pressure in the Wt animal, but not in the N-KO animal.

Further, on the contrary, candidate substances can be screened for a substance having an action for lowering blood pressure without blocking the influx of Ca²⁺ passed through the N-type Ca channel by selecting a substance having an action for lowering blood pressure in the N-KO animal. Although the N-KO mouse of the present invention had been expected to be deficient in blood pressure control through nervous systems, the average blood pressure of the N-KO mice was higher than that of the Wt animals and this suggested that a blood pressure control system through an endogenous factor intensely operated in the N-KO animal. Therefore, the N-KO animal is particularly useful for screening for a substance having an action for lowering blood pressure through an endogenous factor.

Specifically, for example, when an N-KO mouse and a wild-type mouse (hereinafter, referred to as Wt mouse) are used, following anesthetization of each mouse, a tube is placed in the trachea and artificial respiration is attained by using an animal ventilator with air ventilation of 0.2 ml at a respiratory frequency of 140 breaths/min. A polyethylene tube filled with a physiological saline containing heparin is inserted into the right common carotid artery and connected to a pressure transducer to measure the blood pressure. Each of candidate substances to be subjected to the screening is administered by using an indwelling catheter placed in the left common carotid artery, and a substance having an action for lowering the blood pressure is selected from the candidates.

### 2. Method for screening for substance having analgesic action (analgesic drug)

Candidate substances can be screened for a substance having an analgesic action through or not through blocking of the influx of Ca²⁺ passed through N-type Ca channel by administering the candidate substances to an N-KO animal and a Wt animal and comparing their analgesic actions. The analgesic action can be confirmed by, for example, a formalin test, hot-plate test, acetic acid-induced writhing test, tail-flick test, tail-pinch test or the like.

Specifically, for example, in the case of a formalin test using an N-KO mouse and a Wt mouse, 20 µl of 3% formalin is subcutaneously administered to each of the N-KO mouse and the Wt mouse at the sole of the left hind leg. Then, the duration of the mouse's behavior of licking its left hind leg (licking) was measured over 30 minutes for use as an indicator of pain. Substances subjected to the screening are administered, and a substance reducing the pain indicator can be selected.

### 3. Method for screening for substance having action for lowering blood sugar level (hypoglycemic drug)

Candidate substances can be screened for a substance having a hypoglycemic action through or not through blocking of the influx of Ca²⁺ passed through N-type Ca channel by administering each of the candidate substances to an N-KO animal and a Wt animal and comparing their hypoglycemic actions.

Specifically, for example, when an N-KO mouse and a Wt mouse are used, blood is collected from the caudal vein of each of the N-KO mouse and the Wt mouse under a fed condition (fasted for 2 hours prior to blood collection) or a fasted condition (fasted for 18 hours) and the blood sugar level is measured. The blood sugar level can be measured, for example, as follows. 10 µl of blood and 90 µl of 0.6 N perchloric acid are mixed and subjected to centrifugation (7,000 rpm, 2 min). Then, 20 µl of the supernatant and 300 µl of color developing solution of Glucose CII-Test Wako (Wako Pure Chemical Industries) are mixed and allowed to react at 37°C for 5 minutes, and absorption of the reaction mixture is measured at 505 nm.

A drug containing a substance having a pharmacological action as an active ingredient can be manufactured according to a usual drug preparation method. The drug may be a pharmaceutical composition of a substance having a pharmacological action and a pharmaceutically acceptable carrier.

### Examples

The present invention will be explained more specifically with reference to the following examples. However, the scope of the present invention is not limited to these examples.

### Example 1: Disruption of gene coding for N-type Ca channel by gene targeting

### (1) Cloning of gene coding for N-type Ca channel α_{1B} subunit

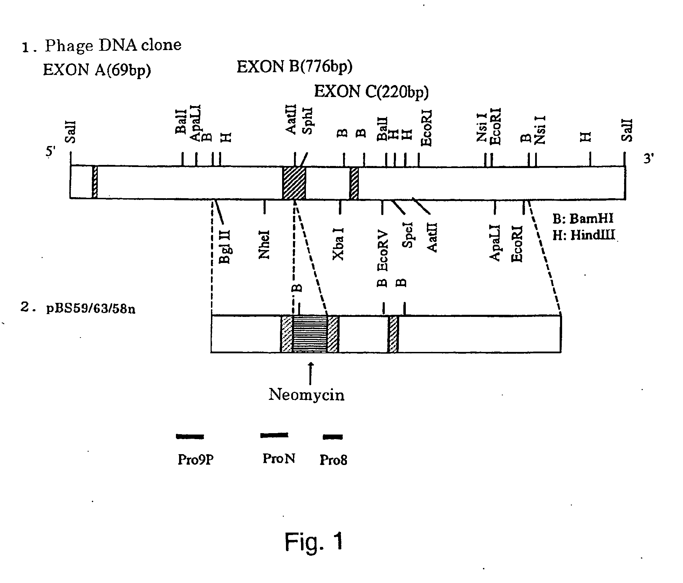

Primers (SEQ ID NOS: 4 and 5) were designed based on the nucleotide sequence of the mouse α_{1B} subunit gene described in FEBS Letters, 338, pp.1-5, 1994, and PCR was performed by using the mouse cDNA library as a template to obtain DNA having the nucleotide sequence of SEQ ID NO: 6. By using this DNA as a probe, a phage DNA clone with a part of the gene coding for the N-type Ca channel α_{1B} subunit was isolated from a 129SVJ-derived mouse genomic library (λFIXII). The restriction enzyme map of the obtained phage DNA clone is shown in Fig. 1.

### (2) Construction of targeting vector

A targeting vector was prepared by a method wherein a region including exon B in the α_{1B} subunit gene was used as a homologous gene region; a neomycin resistance gene was introduced into the exon B (Fig. 1), and the thymidine kinase gene of herpes simplex virus was introduced as a negative selection gene (Suzanne, L. et al., Nature, 336, p.348, 1988).

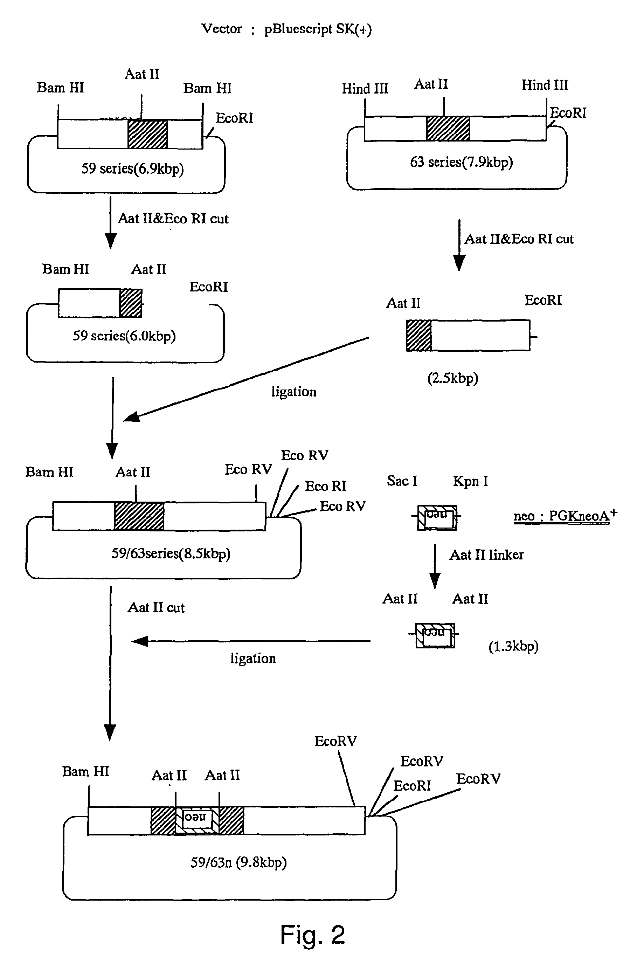

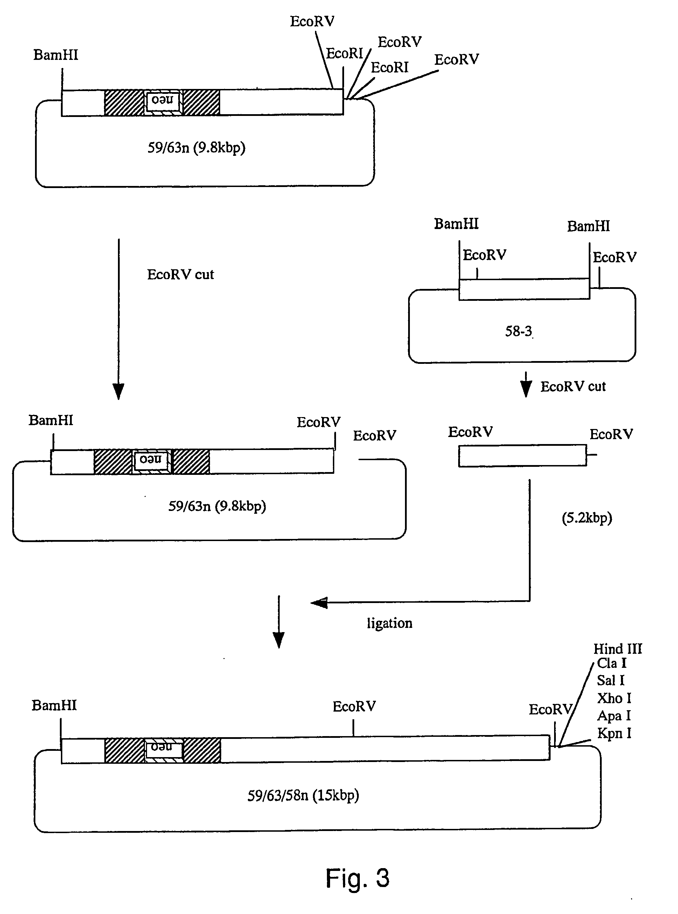

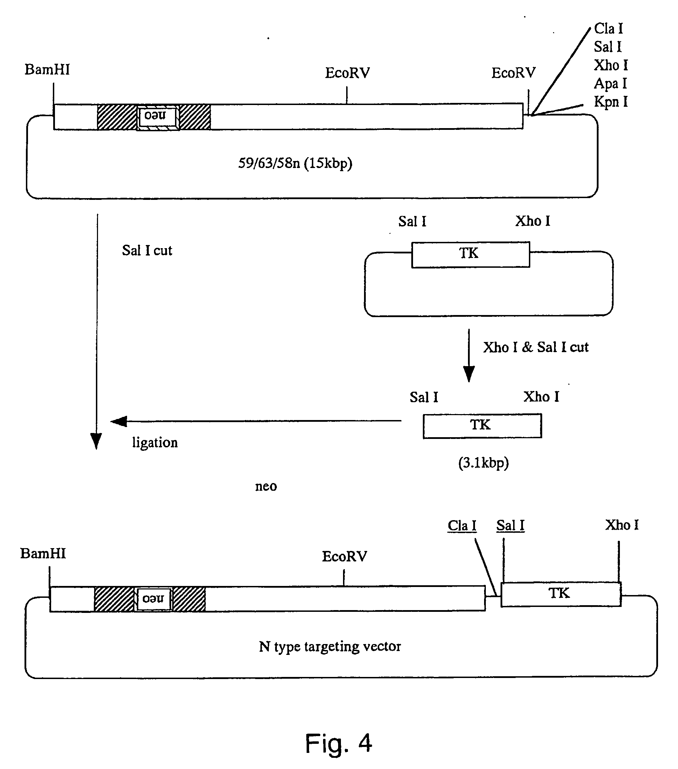

Outline of the construction is shown in Figs. 2-4. The phage DNA clone obtained in (1) was digested with *Bam*HI and subcloned into pBluescript II SK+ to obtain pBS59 and pBS58 having the fragments shown in Figs. 2 and 3. The phage DNA clone was also digested with *Hin*dIII and subcloned into pBluescript II SK+ to obtain pBS63 having a fragment shown in Fig. 2. The pBS59 was digested with *Aat*II and *Eco*RI and a fragment excised from pBS63 with *Aat*II and *Hind*III was introduced thereto to obtain pBS59/63. This pBS59/63 was digested with AatII and a fragment including the neomycin resistance gene was introduced thereto to prepare pBS59/63n. This was further digested with *Eco*RV and a fragment excised from pBS58 with *Eco*RV wa introduced thereto to prepare pBS59/63/58n. The thymidine kinase gene, which is a selection gene, was introduced into the *SalI-XhoI* site of a multicloning site in pBS59/63/58n to produce a targeting vector.

### (3) Acquisition of embryonic stem cell (ES cell) having undergone homologous recombination

The targeting vector obtained in (2) was digested with NotI to form linear DNA (1 mg/ml). As a mouse ES cell, TT2 was used (Yagi, T. et al., Analytical Biochem. 214, p.70, 1993). The linear targeting vector (200 µg/ml) was transfected into the ES cells (1 x 10⁷ cells/ml) by electroporation (250 V, 975 µF, room temperature), and the cells were cultured in a medium containing G418 (250 µg/ml) and ganciclovir (0.2 µM) for 3 days from day 2 of culture, and then cultured in a medium containing G418 (250 µg/ml) for 3 days. DNA was extracted from a part of the generated ES cell colonies, and PCR was performed by using this DNA as a template, and DNA having the nucleotide sequence (SEQ ID NO: 7) outside the targeting vector and DNA having the nucleotide sequence (SEQ ID NO: 8) included in the introduced gene (neomycin resistance gene) as primers. Clones generating 3.7-kb PCR product were assumed as candidates that have possibility of having undergone homologous recombination.

Among the candidate clones, a clone having undergone only homologous recombination was identified by Southern blotting analysis. The extracted genome was digested with *Apa*LI and *Bal*I, hybridized with a probe Pro9P outside the targeting vector (about 0.9-kbp DNA 5'-upstream from the homologously recombined region was obtained by PCR, see Fig. 1) and a probe Pro8 inside the targeting vector (about 0.8-kbp DNA excised from pBS59 with *Sph*I and *Bam*HI, see Fig. 1). A clone having undergone homologous recombination was selected, that is, a clone was selected for which a 6.9-kb band was detected in the *Apa*L1 digestion product and a 4.6-kb band was detected in the *Bal*I digestion product when Pro9P was used as a probe, while a 6.9-kb band was detected in the *Apa*L1 digestion product and a 2.4-kb band was detected in the *Bal*I digestion product when Pro8 was used as a probe.

### (4) Preparation of N-KO mouse

To a female mouse, 5 IU of pregnant mare's serum gonadotropin (PMSG, Serotropin, Teikoku Hormone Mfg., Tokyo) and 2.5 IU of human chorionic gonadotropin (hCG, Gonatropin, Teikoku Hormone Mfg., Tokyo) were intraperitoneally administered. On day 2.5 after fertilization, an 8-cell embryo was obtained by the oviduct-uterus perfusion method.

To the 8-cell embryo, the ES cells having undergone homologous recombination obtained in (3) were microinjected under an inverted microscope (DIAPHOTO TMD, Nippon Kogaku Kogyo, Tokyo) by using a micromanipulator (coarse-adjustment electric manipulator equipped with a suspended type joystick three-dimensional oil hydraulic micromanipulator, Narishige, Tokyo), a microinjector (Narishige, Tokyo), an injection pipette and a holding pipette. Further, as an injection dish, there were used several 5-µl medium droplets containing floating ES cells formed on Falcon 3002 (Becton Dickinson Labware) and overlaid with liquid paraffin.

Vasoligated male mice and normal female mice were mated to prepare pseudopregnant mice, and manipulated eggs into which three different ES cell clones having undergone homologous recombination were microinjected were implanted in the pseudopregnant mice. The pseudopregnant mice were generally anesthetized with 50 mg/kg body weight of pentobarbital sodium (Nembutal, Abbott Laboratories). Then, both flanks were incised about 1 cm to expose the ovary and the oviduct. The bursa ovarica was incised by using tweezers under a stereoscopic microscope to expose the fimbriae tubae. Subsequently, 7 to 8 manipulated eggs per oviduct were transferred into the fimbriae tubae. Then, the oviduct and the ovary were returned to the abdominal cavity, and both the incision sites were sutured.

The mice in which the manipulated eggs were implanted to be pregnant delivered a 100% chimeric mouse with a black coat color. To confirm that germ cells of the obtained 100% chimeric mouse were derived from the ES cells, the chimeric mouse was mated with an ICR female mouse, and their offspring mice were examined. The coat color of all the offspring mice was black, and hence it was confirmed that the germ cells of the chimeric mouse were derived from the ES cells. He mice were obtained by mating the chimeric mouse with C57BL/6, and an N-KO mouse was obtained by mating He mice with each other.

The genotypes of the obtained mice were confirmed based on differences in size of DNA fragments generated by PCR. The tail of each mouse was excised in a length of about 2-3 mm and digested (55°C, 2 hours) with a proteinase K solution (lysis buffer (Perkin Elmer) was diluted two-fold with PBS(-), 1% mercaptoethanol, 0.25 mg/ml of proteinase K). Thereafter, genomic DNA was extracted by a usual method and dissolved in 100-200 µl of distilled water to prepare a template for PCR. Primers were designed for the sequence included in the neomycin resistance gene (SEQ ID NO: 8) and two sites in the α_{1B} subunit gene (SEQ ID NOS: 9 and 10), and PCR was performed to identify the genotype of each individual. The gene having undergone a mutation produced a 520-bp PCR product, whereas the wild-type gene produced a 490-bp PCR product.

As required, the genotype was also confirmed by Southern blotting analysis. When the genomic DNA extracted from the mouse tail was digested with *Bam*HI, and a region adjacent to the neomycin resistance gene in the targeting vector was hybridized with a probe ProN (about 1-kbp DNA excised from pBS59 with NcoI, see Fig. 1), only a 3.1-kb band was detected for the N-KO mouse.

The expression amount of mRNA in mouse brain was confirmed by Northern blotting. Total RNA was extracted from each of brains of 3 mice having each genotype by the AGPC method. Purified mRNA was obtained from the total RNA by using an oligo dT column (Amersham Pharmacia Biotech). The mRNA (5 µg/lane) was subjected to electrophoresis on 0.5% gel and hybridized with DNA having the nucleotide sequence of SEQ ID NO: 6 as a probe. The Northern blotting analysis showed that the mRNA expressed in the Wt mouse had completely disappeared in the N-KO mouse.

### (5) Confirmation of N-KO based on electric current passed through N-type Ca channel

By using nerve cells in the dorsal root ganglia of the Wt mouse and the N-KO mouse, changes in the amount of Ca²⁺ influx inhibited by the ω-conotoxin GVIA were measured by using Ba²⁺ as a charge carrier by the whole-cell patch clamp method.

A 5- to 8-week old mouse was anesthetized with ether, and its dorsal root ganglia were removed and digested in a Krebs solution by using pronase (0.2 mg/ml) first for 30 minutes and then thermolysin (0.2 mg/ml) for 30 minutes to isolate cells.

A patch clamp amplifier (Axopatch 200B) was set at a whole cell mode, and measurement was performed at room temperature. A patch pipette (outer diameter: 1.5 mm, inner diameter: 1.1 mm) was prepared by using a P-87 Flaming-Brown micropipette puller (Sutter Instrument). A solution containing 3 mM BaCl₂, 155 mM tetraethylammonium chloride, 10 mM HEPES and 10 mM glucose (pH 7.4) was used as the outer solution of the isolated nerve cells and the patch pipette was filled with a solution containing 85 mM cesium aspartate, 40 mM CsCl, 2 mM MgCl₂, 5 mM EGTA, 2 mM ATPMg, 5 mM HEPES and 10 mM creatine phosphate (pH 7.4). The electric resistance of the pipette was 1-2 Mohm, and the current of Ba²⁺ obtained by a stimulus at 100 kHz was analyzed by using pCLAMP (Axon Instruments). The currents of the total Ca channels of nerve cells were measured for the Wt mouse and the N-KO, and the ω-conotoxin GVIA (1 µM) was added to measure the current other than that for N-type Ca channel.

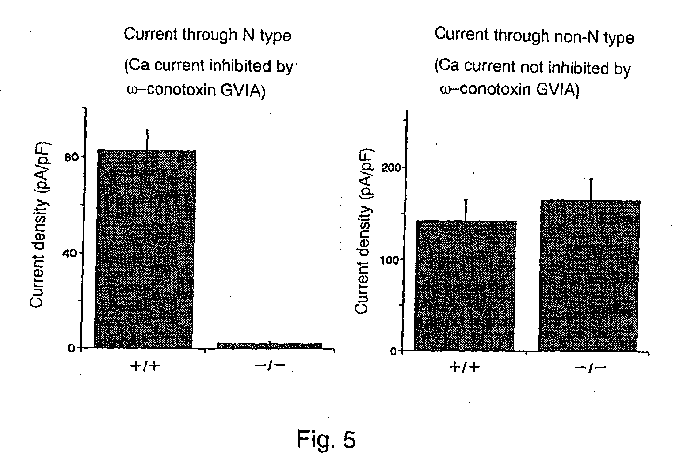

The results are shown in Fig. 5. The values in the figure are average values, and the bars represent standard deviations (Wt (+/+): n = 4, N-KO (-/-): n = 7). It was electrophysiologically proved that the influx of Ca²⁺ inhibited by the ω-conotoxin GVIA was not observed in nerve cells of the nerve dorsal root ganglia extracted from the N-KO mouse, and thus it was confirmed that the N-KO mouse lacked functional N-type Ca channel.

### (6) Comparison of body weight, heart rate and blood pressure between genotypes

Body weight, heart rate and average blood pressure of a Wt mouse and those of an N-KO mouse were compared and examined. The Wt mice (15- to 16-week old, male, n = 4) and the N-KO mice (15- to 16-week old, male, n = 4) were anesthetized with 10% urethane. Following tracheal intubation, artificial respiration was performed by using an animal ventilator (Columbs) with a ventilation volume of 0.2 ml at a respiratory frequency of 140 breaths/min. A polyethylene tube filled with physiological saline containing heparin was inserted into the right common carotid artery and connected to a pressure transducer (Millar, Model MPC-500) to measure the blood pressure. The heart rate was obtained from blood pressure pulsation.

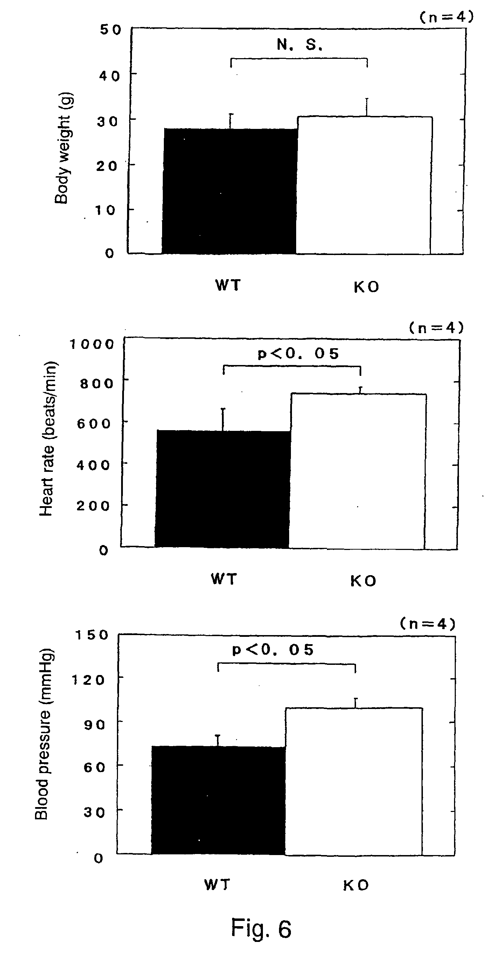

The results are shown in Fig. 6. The values in the figure are average values, and the significant differences were determined by the t-test. There was no difference in body weight between the two groups (28.2 ± 3.2 g vs. 30.8 ± 4.0 g). The heart rate and the average blood pressure of the N-KO mice were significantly higher than those of the Wt mice (562 ± 101.9 beats/min vs. 742 ± 32.5 beats/min, p < 0.05, 73.4 ± 7.7 mmHg vs. 100.0 ± 6.6 mmHg, p < 0.05).

These results are considered to suggest a possibility that the heart rate and the blood pressure were maintained at a constant level due to vagotonia in the Wt mouse, whereas a vagotonia state was lost due to the lack of sympathetic innervation and parasympathetic innervation, and the heart rate and the blood pressure were significantly higher in the N-KO mouse. Further, it is also considered to be possible that the N-KO mouse constantly has higher levels of factors involved in the pressure rise such as nerve transmitter substances including noradrenaline, angiotensin II, endothelin etc.

### Example 2: Changes in blood pressure upon administration of ω-conotoxin GVIA to mouse

Changes in heart rate and blood pressure of a Wt mouse and those of an N-KO mouse due to the ω-conotoxin GVIA were evaluated. Wt mice (15- to 16-week old, male, body weight 28.2 ± 3.2 g, n = 4) and N-KO mice (15- to 16-week old, male, body weight 30.8 ± 4.0 g, n = 4) were anesthetized with 10% urethane. Following tracheal intubation, artificial respiration was performed by using an animal ventilator (Columbs) with a ventilation volume of 0.2 ml at a respiratory frequency of 140 breaths/min. A polyethylene tube filled with physiological saline containing heparin was inserted into the right common carotid artery and connected to a pressure transducer (Millar, Model MPC-500) to measure the blood pressure. Further, a catheter was indwelled in the left common carotid artery to administer ω-conotoxin GVIA (omega-CgTx GVIA, 30 µg/kg).

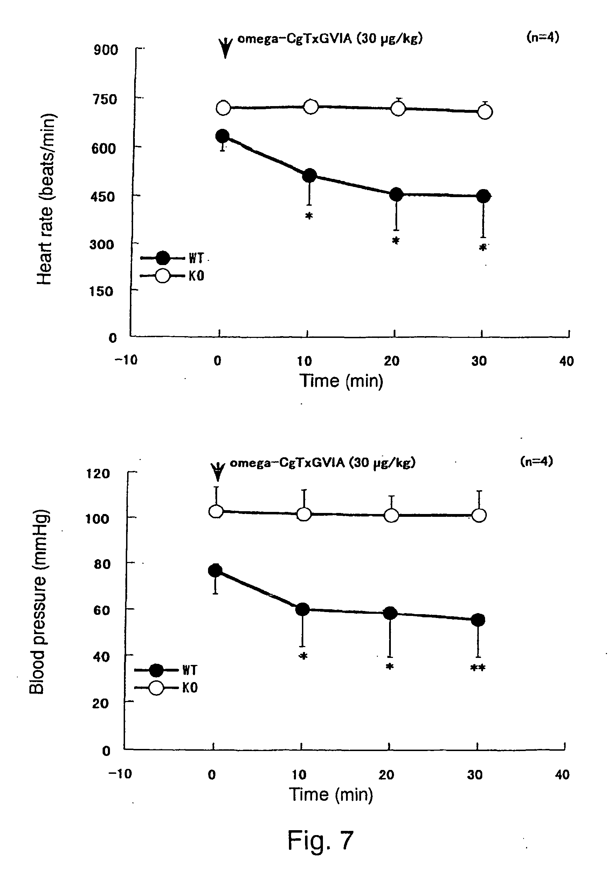

The results are shown in Fig. 7. The values in the figure are average values, and the bars represent standard deviations. In the Wt mice, significant decreases in the heart rate and the blood pressure were observed from 10 minutes after the administration. On the other hand, no changes in the heart rate and the blood pressure were observed in the N-KO mouse even after the administration of ω-conotoxin GVIA.

These results suggest that N-type Ca channel should be involved in the controls of heart rate and blood pressure. Therefore, it is considered that the N-KO mouse is an animal model useful for elucidating the control mechanisms of heart rate and blood pressure.

### Example 3: Experiment about blood pressure control mechanism - Examination of blood pressure change with bilateral carotid occlusion

Blood pressure changes with bilateral carotid occlusion (henceforth referred to as BCO) in a Wt mouse and an N-KO mouse were evaluated. Wt mice (15-to 16-week old, male, body weight 28.2 ± 3.2 g, n = 4) and N-KO mice (15- to 16-week old, male, body weight 30.8 ± 4.0 g, n = 4) were anesthetized with 10% urethane. Following tracheal intubation, artificial respiration was performed by using an animal ventilator (Columbs) with a ventilation volume of 0.2 ml at a respiratory frequency of 140 breaths/minute. A polyethylene tube filled with physiological saline containing heparin was inserted into the right common carotid artery and connected to a pressure transducer (Millar, Model MPC-500) to measure the blood pressure. Further, a silk thread (Natsume, suture needle with thread, Black broad silk No. 8-0) for artery occlusion was placed on the left common carotid artery, and the blood flow was transiently stopped by holding the silk thread upward to obtain a BCO state.

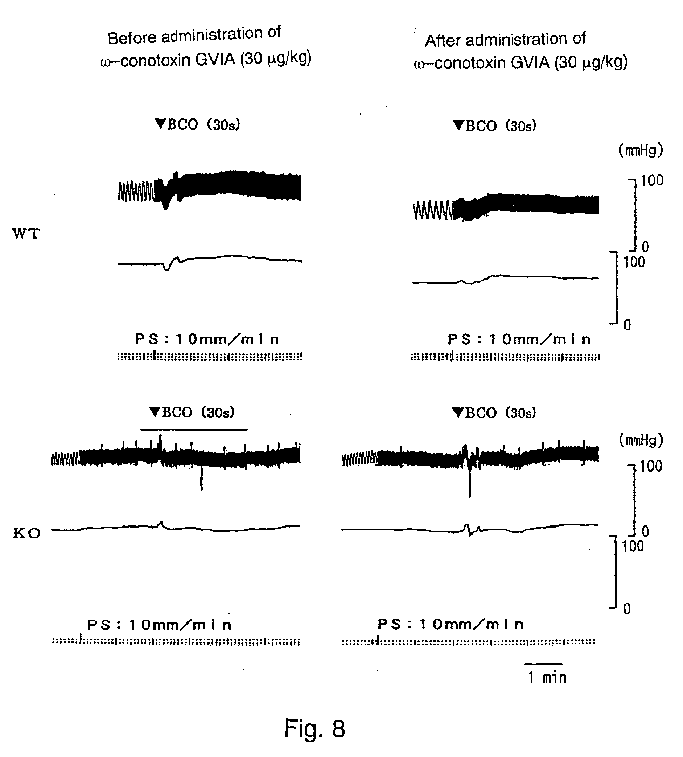

As a result of BCO for 30 seconds, a transient rise of blood pressure was observed in the Wt mice, but this blood pressure rise mostly disappeared after the administration of ω-conotoxin GVIA (30 µg/kg). On the other hand, no blood pressure rise was observed in the N-KO mice even in the BCO state. Typical data are shown in Fig. 8 (upper lines: arterial pressure, lower lines: average blood pressure in Fig. 8).

From these results, it is considered that the N-KO mouse lacked a pressure reflex mechanism through a pressure receptor present in the internal carotid artery, and that nerve transmitter substances were not released at least from a neuroterminal of the sympathetic nerve postganglionic fiber.

It is considered that roles of Ca channel of each subtype at an autonomic neuroterminal involved in the cardiocirculatory control mechanism can be revealed by using the N-KO mouse.

### Example 4: Examination of analgesic effect on formalin administration

In this experiment, a Wt mouse, He mouse and N-KO mouse (male, 6-week old) were used. 30 µl of formaldehyde solution (WAKO, 35.0-38.0%, first grade, Lot No. DLL4284) was added to 970 µl of physiological saline. This is referred to as 3% formalin. 20 µl of the 3% formalin was subcutaneously administered to the mouse at the sole of left hind leg. After the formalin was administered, the duration of the mouse's behavior of licking its left hind leg (licking) was measured over 30 minutes for use as an indicator of pain. The duration was summed up every 5 minutes and represented in seconds. The significant difference was obtained by performing a parametric one-way layout variance analysis and then Dunnet's multiple comparison test (*: 0.01(p<0.05, **: p < 0.01 vs. control group). In the test, a statistical analysis support system into which SAS 6.12 (SAS Institute Japan, Tokyo) was incorporated was used.

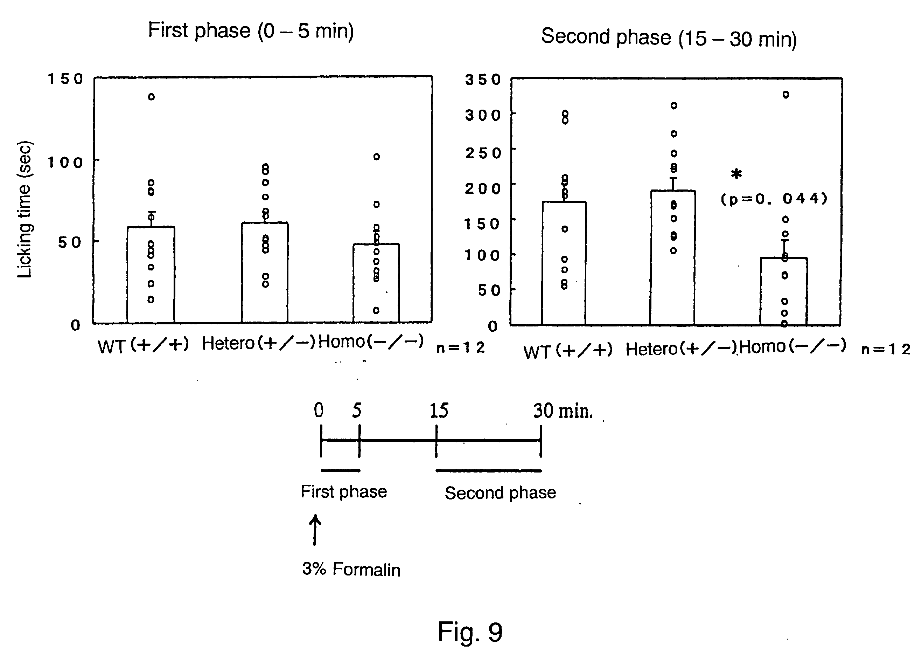

As a result, no difference was observed for pain in a first phase (0-5 minutes) in the N-KO mouse compared with the Wt mouse and the He mouse, but an analgesic effect was observed on pain in a second phase (15-30 minutes) (Fig. 9). This suggests that N-type Ca channel is involved in transmission of pain. It is also suggested that, since the transmission of pain is not completely suppressed, the N-KO mouse is useful for evaluation of analgesic drugs through action points other than N-type Ca channel.

### Example 5: Blood sugar level of N-KO mouse

### 1. Measurement of blood sugar level of N-KO mouse

10 µl of blood was collected from each caudal vein of N-KO mice and Wt mice (Wt (+/+) male: n = 9, Wt female: n = 10, N-KO (-/-) male: n = 10, N-KO female: n = 10) under a fed condition (fasted for 2 hours prior to blood collection) or a fasted condition (fasted for 18 hours), mixed with 90 µl of 0.6 N perchloric acid and centrifuged (7,000 rpm, 2 min). 20 µl of the supernatant and 300 µl of a color developing solution of Glucose CII-Test Wako (Wako Pure Chemical Industries) were mixed on a 96-well microplate, and allowed to react at 37°C for 5 minutes, and absorbance of the mixture was measured at 505 nm.

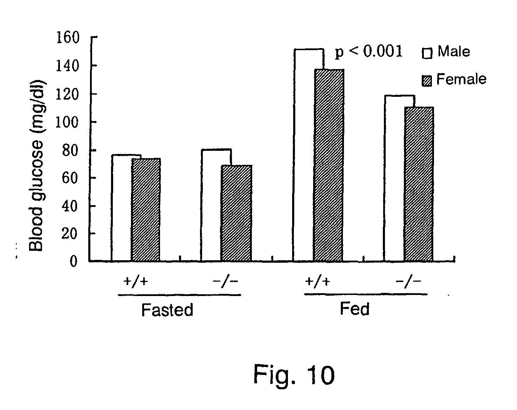

The results are shown in Fig. 10. The values in the figure are average values. In the case of the fed condition, the N-KO mice showed significantly low blood sugar levels compared with those of the Wt mice (t-test). On the other hand, under the fasted condition, no significant difference was observed between blood sugar levels of those mice.

These results show that blood sugar level can be raised by activation of nerve transmission through the N-type Ca channel and indicate that the N-type Ca channel should be involved in normalization of blood sugar level (maintenance of homeostasis).

### 2. Glucose tolerance test of N-KO mouse

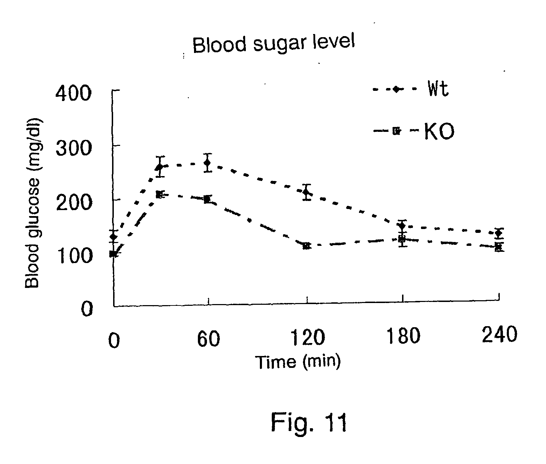

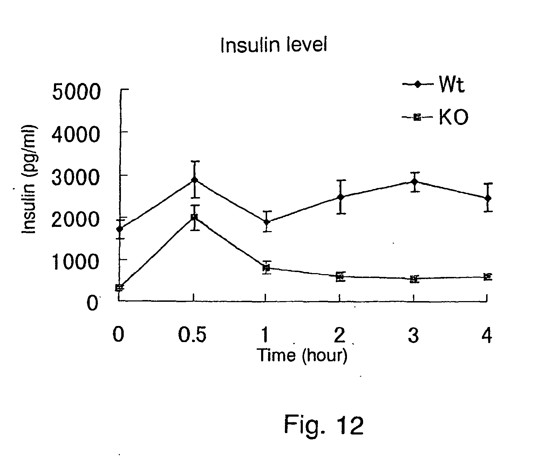

Wt mice and N-KO mice (male, 9- to 10-month old, Wt: n = 9, N-KO: n = 9 for determination of blood sugar level, Wt: n = 8, N-KO: n = 9 for determination of insulin level) that had fasted for 16 hours were orally administered with 2 g/kg body weight of 20% glucose solution, and 10 µl each of blood was collected from the caudal vein after 0, 0.5, 1, 2, 3 and 4 hours to measure blood sugar level by the same method as described above. Further, 10 µl of blood collected in the same manner was mixed with 10 µl of heparin-containing physiological saline and centrifuged, and then the insulin level in the supernatant was quantified by using an enzyme immunoassay kit (Morinaga Milk Industry Co., Ltd, Biochemical Research Laboratory). The blood sugar levels and the insulin levels are shown in Figs. 11 and 12, respectively. In Figs. 11 and 12, the values are average values, and the bars represent standard deviations.

As shown in Fig. 11, the fasting blood sugar levels of the N-KO mice were significantly lower than those of the Wt mice, and the blood sugar levels changed within a significantly low value range even after glucose was administered. It was considered that the 9- to 10-month old Wt mice had age-related insulin resistance, whereas changes in the blood sugar levels of the N-KO mice were similar to those of young mice.

This difference was also shown in the insulin levels shown in Fig. 12, and the Wt mice maintained a high insulin concentration before and after the glucose administration, whereas the N-KO mouse showed a low insulin concentration, which returned to the level before the glucose administration after 1 hour. Further, the insulin levels of the Wt mice significantly varied depending on each individual.

These experimental results indicate that the N-KO mouse does not become insulin resistant easily, and N-type Ca channel is involved in insulin resistance and further indicate that activation of the N-type Ca channel is associated with normalization of blood sugar level.

Amounts of glucagon and leptin were also measured, but no difference was observed between the Wt mice and the N-KO mice.

### 3. Immunofluorescence staining of spleen

In order to further confirm the involvement of N-type Ca channel in insulin resistance, pancreatic β cells in islets of Langerhans of a Wt mouse and an N-KO mouse were compared.

A Wt mouse and an N-KO mouse (male, 11-month old) were anesthetized with Nembutal and subjected to abdominal section, then a portion around a valve of the right atrium was excised, and blood was removed. PBS containing heparin (4 U/ml) was injected from the left ventricle, and whitening of the liver was confirmed. Then, 4% paraformaldehyde dissolved in PBS was further injected. When rigor of each individual was confirmed, the pancreas was removed and fixed with 4% paraformaldehyde at 4°C for 1 hour. Following the fixation, the pancreas was left overnight in PBS containing 30% sucrose at 4°C and embedded in an OCT compound to prepare a thin section.

The thin section was stained by using a guinea pig anti-insulin serum (Linco Research) as primary antibodies and rhodamine-labeled anti-guinea pig IgG antibodies (Chemicon International) as secondary antibodies, and the β cells containing insulin were observed with a fluorescence microscope. Similarly, the thin section was stained by using rabbit anti-glucagon antibodies (Linco Research) and FITC-labeled anti-rabbit IgG antibodies (Organon Teknika), and the α cells containing glucagon were observed.

In the N-KO mouse, a cell aggregation of β cells was small, and an increase in the number of β cells with aging was not observed, which was observed in the Wt mouse. On the other hand, no difference was observed in α cells between the both mice.

It is considered that the Wt mouse had age-related insulin resistance and insulin production in β cells was accelerated, while the N-KO mouse did not have insulin resistance.

### Example 6: Autonomic innervation of atrial muscle contractile force of N-KO mouse

Autonomic innervation of atrial muscle contractile force of an N-KO mouse was examined. The atriums were isolated from mice (Wt mice and N-KO mice: n = 5 each), and the contractile force and the action potential were simultaneously recorded by giving a direct muscle stimulus and a nerve stimulus from two of stimulators. As for stimulus conditions, the basal stimulus was given with a frequency of 2 Hz, a voltage just above the threshold and a pulse width of 1 msec. The nerve stimulus was given with a frequency of 200 Hz, a voltage 1.5 times as high as the basal stimulus and a pulse width of 0.1 msec. Four nerve stimuli per basal stimulus were given during a refractory period of the cardiac muscle, which lasted 15 seconds.

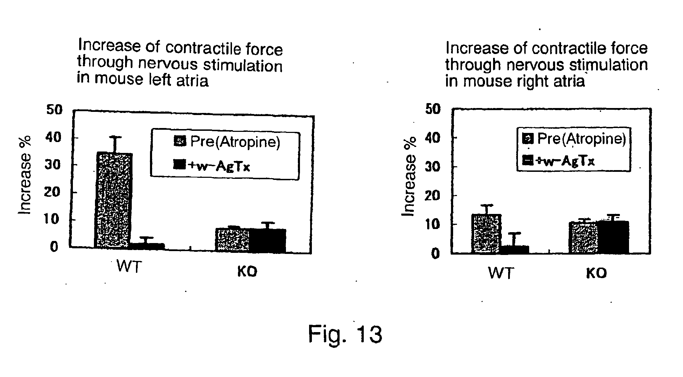

Fig. 13 shows experimental results in the left atrium and the right atrium of 5 cases. The values are average values, and the bars represent standard deviations. In the figure, w-AgTx represents ω-agatoxin GIVA.

In the left atrium, the atrial muscle contractile force of the Wt mouse was greatly increased by the nerve stimulus in the presence of atropine, and this increase in the contractile force was almost completely inhibited by 30 nM ω-conotoxin GVIA (ω-agatoxin GIVA). On the other hand, although a slight increase was observed in the atrial muscle contractile force of the N-KO mouse by the nerve stimulus in the presence of atropine, this increase in the contractile force was not suppressed by the ω-conotoxin GVIA up to 100 nM. These increases in the contractile force were completely inhibited by 0.1 µM tetrodotoxin, although the data are not shown in the figure.

Although no increase in the contractile force of the atrial muscle caused by the atropine nerve stimulus was not so remarkable in the right atrial muscle as in the left atrium, the obtained result was almost similar to that of the left atrium.

These results are considered to suggest that the release of norepinephrine (NE) from the sympathetic nerve mostly depended on N-type Ca channel in the Wt mouse, but Ca channels of other types were increased in a compensatory manner and contributed to the release of NE in the N-KO mouse. In the Wt mouse, it is expected that an increase in the contractile force by a nerve stimulus in the right atrium is smaller than that in the left atrium and hence there is a difference in innervation densities in the left and right atriums.

### Industrial Applicability

The present invention provides an animal that does not show a functional expression of N-type Ca channel. By using the animal of the present invention, the function of the N-type Ca channel can be deduced. Further, by administrating a drug to an N-KO animal and a Wt animal, whether the drug acts on the N-type Ca channel can be deduced from the difference in their responses. Furthermore, there are provided a method for screening for a substance having a pharmacological action on blood pressure control, transmission of pain, blood sugar level control and so forth by using the animal of the present invention

## Claims

1. A mouse in which a gene coding for α_{1B} subunit of the N-type calcium channel is disrupted so that the mouse lacks functional N-type calcium channel.

2. The mouse according to Claim 1, wherein the gene comprises DNA defined in the following (a) or (b):
a) DNA which comprises the nucleotide sequence of SEQ ID NO: 1;
b) DNA which is hybridizable with DNA comprising the nucleotide sequence of SEQ ID NO: 1 under a stringent condition and codes for an α_{1B} subunit of functional N-type calcium channel.

3. A method for determining an action of a substance, which comprises steps of administering a substance to the mouse as defined in any of Claims 1-2 and determining an action of the substance on the mouse.

4. A method for determining an action of a substance, which comprises steps of administering a substance to the mouse as defined in any one of Claims 1-2 and a wild-type mouse, and comparing actions of the substance on the mouse according to any one of Claims 1-2 and the wild-type mouse to determine the action of the substance on the N-type calcium channel.

5. A method for screening for a substance having a pharmacological action, which comprises a step of determining pharmacological action of the substance by the method as defined in Claim 3 or 4.

6. The method according to Claim 5, wherein the pharmacological action is an action for lowering blood pressure.

7. The method according to Claim 5, wherein the pharmacological action is an analgestic action.

8. The method according to Claim 5, wherein the pharmacological action is an action for lowering blood sugar level.

## Patentansprüche

1. Maus, bei der ein Gen, das eine α_{1B} Untereinheit des N-Typ Calciumkanals kodiert, unterbrochen ist, so dass die Maus keinen funktionellen N-Typ Calciumkanal aufweist.

2. Maus nach Anspruch 1, worin das Gen eine DNA aufweist, die in der folgenden Weise (a) oder (b) definiert ist:
a) DNA, die die Nukleotidsequenz SEQ ID NO: 1 aufweist,
b) DNA, die mit einer DNA hybridisierbar ist, die die Nukleotidsequenz SEQ ID NO. 1 unter einer strengen Bedingung aufweist, und eine α_{1B} Untereinheit des funktionellen N-Typ Calciumkanals kodiert.

3. Verfahren zur Bestimmung einer Wirkung einer Substanz, das die Schritte des Verabreichens einer Substanz an die im Anspruch 1 oder 2 definierte Maus und des Bestimmens einer Wirkung der Substanz auf die Maus umfasst.

4. Verfahren zur Bestimmung einer Wirkung einer Substanz, die die Schritte des Verabreichens einer Substanz an die im Anspruch 1 oder 2 definierte Maus und an eine frei lebende Maus und des Vergleichens der Wirkungen der Substanz auf die Maus gemäß Anspruch 1 oder 2 und die frei lebende Maus, um die Wirkung der Substanz auf den N-Typ Calciumkanal zu bestimmen, umfasst.

5. Verfahren für ein Screening bezüglich einer Substanz, die eine pharmakologische Wirkung aufweist, das einen Schritt des Bestimmens der pharmakologischen Wirkung der Substanz mittels des in den Ansprüchen 3 oder 4 definierten Verfahrens aufweist.

6. Verfahren nach Anspruch 5, worin die pharmakologische Wirkung eine Wirkung zur Senkung des Blutdrucks ist.

7. Verfahren nach Anspruch 5, worin die pharmakologische Wirkung eine analgetische Wirkung ist.

8. Verfahren nach Anspruch 5, worin die pharmakologische Wirkung eine Wirkung zur Senkung des Blutzuckerspiegels ist.

## Revendications

1. Souris dans laquelle un génécode pour la sous-unité α_{1B} du canal calcique de type N est soumis à une disruption pour que la souris ne dispose pas d'un canal calcique de type N fonctionnel

2. Souris selon la revendication 1,
dans laquelle
le gène comprend ADN défini comme suit par (a) ou (b) :
(a) ADN comprenant la séquence de nucléotides selon SEQ ID NO: 1;
(b) Un ADN qui peut être hybridé avec un ADN comprenant la séquence de nucléotides de SEQ ID NO. 1 sous une condition stricte et codé pour une sous-unité α_{1B} du canal calcique de type N fonctionnel.

3. Procédé pour déterminer une action d'une substance comprenant les étapes consistant à:
- administrer une substance à la souris telle que définie par l'une des revendications 1 et 2,
- déterminaison de l'action de la substance sur la souris.

4. Procédé de détermination de faction d'une substance comprenant les étapes consistant à:
- administrer une substance à la souris telle que définie par l'une des revendications 1 et 2, et une souris de type sauvage, et
- comparaison des actions de la substance sur la souris selon l'une des revendications 1 et 2, et la souris de type sauvage pour déterminer l'effet de la substance sur le canal de calcique de type N.

5. Procédé de criblage d'une substance ayant une action pharmacologique et comprenant l'étape consistant à déterminer l'action pharmacologique de la substance selon le procédé de l'une des revendications 3 ou 4.

6. Procédé selon la revendication 5,
selon lequel
l'action pharmacologique est une action de diminution de la pression sanguine.

7. Procédé selon la revendication 5,
selon lequel
l'action pharmacologique est une action analgésique.

8. Procédé selon la revendication 5,
selon lequel
l'action pharmacologique est une action de diminution du niveau de sucre dans le sang.


### Sequence Listing

<110> Eisai Co., Ltd.
<120> N-TYPE CALCIUM CHANNEL KNOCKOUT ANIMAL
<130> 0019WOOP1098
<150> JP 11-303809
   <151> 1999-10-26
<150> JP 2000-37839
   <151> 2000-02-16
<150> JP 2000-261979
   <151> 2000-08-31
<160> 10
<210> 1
   <211> 7185
   <212> DNA
   <213> Mus musculus
<220>
   <221> CDS
   <222> (121)..(6984)
<400> 1
   ggaattcggc tcgaggggcg aggtccaggc agctcgctgc ggctaggcta ggagcccttg   60
   gcgcgccgcg ccctcggtgc cgggccgcgg agcccgggat gctcgcggcg cccgggagtc  120
   atg gtc cgc ttc ggg gac gag cta ggc ggc cgc tat ggg ggc acc ggc    168
   Met Val Arg Phe Gly Asp Glu Leu Gly Gly Arg Tyr Gly Gly Thr Gly
    1               5                  10                  15
   ggc ggg gag cgg gct cgg ggc ggc ggg gcc ggc ggg gcg ggt ggc ccg    216
   Gly Gly Glu Arg Ala Arg Gly Gly Gly Ala Gly Gly Ala Gly Gly Pro
               20                  25                  30
   ggc cag ggg ggt ctg ccg ccg ggc cag cgg gtc ctg tac aag cag tcc    264
   Gly Gln Gly Gly Leu Pro Pro Gly Gln Arg Val Leu Tyr Lys Gln Ser
           35                  40                  45
   att gcg cag cgc gca cgg act atg gcc ctg tac aac ccc atc cca gtc    312
   Ile Ala Gln Arg Ala Arg Thr Met Ala Leu Tyr Asn Pro Ile Pro Val
       50                  55                  60
   aag cag aac tgc ttc acc gtc aac cgc tcg ctc ttc gtc ttc agc gag    360
   Lys Gln Asn Cys Phe Thr Val Asn Arg Ser Leu Phe Val Phe Ser Glu
    65                  70                  75                  80
   gac aac gtc gtc cgc aaa tac gct aag cgc atc acc gaa tgg ccg ccc    408
   Asp Asn Val Val Arg Lys Tyr Ala Lys Arg Ile Thr Glu Trp Pro Pro
                    85                  90                  95
   ttc gaa tac atg atc ctg gcc acc atc atc gcc aac tgc att gtt ctg    456
   Phe Glu Tyr Met Ile Leu Ala Thr Ile Ile Ala Asn Cys Ile Val Leu
               100                 105                 110
   gcc ctg gag cag cac ctc cct gat ggg gac aag act ccc atg tct gag    504
   Ala Leu Glu Gln His Leu Pro Asp Gly Asp Lys Thr Pro Met Ser Glu
           115                 120                 125
   cga cta gat gac acg gag cct tac ttc atc ggg atc ttt tgc ttt gag    552
   Arg Leu Asp Asp Thr Glu Pro Tyr Phe Ile Gly Ile Phe Cys Phe Glu
       130                 135                 140
   gcg ggc atc aag atc ata gcc ctg ggc ttt gtt ttc cac aag ggc tcc    600
   Ala Gly Ile Lys Ile Ile Ala Leu Gly Phe Val Phe His Lys Gly Ser
   145                 150                 155                 160
   tac ctt cgg aac ggc tgg aat gtc atg gac ttc gtg gtg gta ctc acg    648
   Tyr Leu Arg Asn Gly Trp Asn Val Met Asp Phe Val Val Val Leu Thr
                   165                 170                 175
   ggg att ctc gcc aca gct gga act gac ttt gac ctg cgc aca ctg agg    696
   Gly Ile Leu Ala Thr Ala Gly Thr Asp Phe Asp Leu Arg Thr Leu Arg
               180                 185                 190
   gct gtg cgt gtg ctt agg ccc ctg aag ctg gtg tct gga att cca agc    744
   Ala Val Arg Val Leu Arg Pro Leu Lys Leu Val Ser Gly Ile Pro Ser
           195                 200                 205
   ttg cag gtg gtg ctt aag tcc atc atg aag gcc atg gtc ccg ctg ctg    792
   Leu Gln Val Val Leu Lys Ser Ile Met Lys Ala Met Val Pro Leu Leu
       210                 215                 220
   cag att ggg ctg ctg ctc ttc ttt gcc atc ctc atg ttt ggc atc atc    840
   Gln Ile Gly Leu Leu Leu Phe Phe Ala Ile Leu Met Phe Gly Ile Ile
   225                 230                 235                 240
   ggc ctc gag ttc tat atg ggc aaa ttc cat aag gcc tgt ttc ccc aac    888
   Gly Leu Glu Phe Tyr Met Gly Lys Phe His Lys Ala Cys Phe Pro Asn
                   245                 250                 255
   agc aca gat aca gag cct gtg ggt gac ttt ccc tgt ggc aaa gat ccc    936
   Ser Thr Asp Thr Glu Pro Val Gly Asp Phe Pro Cys Gly Lys Asp Pro
               260                 265                 270
   cct gct cgt cag tgt gat ggt gac acc gaa tgc cgg gag tac tgg cca    984
   Pro Ala Arg Gln Cys Asp Gly Asp Thr Glu Cys Arg Glu Tyr Trp Pro
           275                 280                 285
   gga ccc aac ttt ggt atc acc aat ttt gac aac atc ctg ttt gcc atc   1032
   Gly Pro Asn Phe Gly Ile Thr Asn Phe Asp Asn Ile Leu Phe Ala Ile
       290                 295                 300
   ttg aca gtg ttc cag tgt atc acc atg gag ggc tgg act gac atc ctc     1080
   Leu Thr Val Phe Gln Cys Ile Thr Met Glu Gly Trp Thr Asp Ile Leu
   305                 310                 315                 320
   tac aat aca aat gat gcg gct ggc aac acg tgg aac tgg ttg tac ttc     1128
   Tyr Asn Thr Asn Asp Ala Ala Gly Asn Thr Trp Asn Trp Leu Tyr Phe
                   325                 330                 335
   atc ccc ctc atc atc att ggc tcc ttc ttc atg ctc aac ctg gtg ctg     1176
   Ile Pro Leu Ile Ile Ile Gly Ser Phe Phe Met Leu Asn Leu Val Leu
               340                 345                 350
   ggt gtg ctt tct gga gag ttt gcc aag gag cgg gag cga gtc gag aac     1224
   Gly Val Leu Ser Gly Glu Phe Ala Lys Glu Arg Glu Arg Val Glu Asn
           355                 360                 365
   cgc cga gcc ttc ctg aag ctc cgc agg cag cag cag att gag cga gag     1272
   Arg Arg Ala Phe Leu Lys Leu Arg Arg Gln Gln Gln Ile Glu Arg Glu
       370                 375                 380
   ctg aat ggg tac ttg gag tgg atc ttc aag gca gag gaa gtc atg ttg     1320
   Leu Asn Gly Tyr Leu Glu Trp Ile Phe Lys Ala Glu Glu Val Met Leu
   385                 390                 395                 400
   gca gag gag gac aag aat gca gaa gag aaa tcc cct ttg gat gtg ttg     1368
   Ala Glu Glu Asp Lys Asn Ala Glu Glu Lys Ser Pro Leu Asp Val Leu
                   405                 410                 415
   aag aga gct gcc acc aag aag agc cga aat gac ctc atc cat gca gaa     1416
   Lys Arg Ala Ala Thr Lys Lys Ser Arg Asn Asp Leu Ile His Ala Glu
               420                 425                 430
   gag ggg gag gac cgg ttt gta gac ctc tgt gca gtt ggg tct cca ttt     1464
   Glu Gly Glu Asp Arg Phe Val Asp Leu Cys Ala Val Gly Ser Pro Phe
           435                 440                 445
   gct cgt gcc agc ctc aag agt ggg aag acg gag agc tca tcg tac ttc     1512
   Ala Arg Ala Ser Leu Lys Ser Gly Lys Thr Glu Ser Ser Ser Tyr Phe
       450                 455                 460
   cgg aga aag gag aag atg ttc cgg ttc ttt atc cgg cgt atg gtg aaa     1560
   Arg Arg Lys Glu Lys Met Phe Arg Phe Phe Ile Arg Arg Met Val Lys
   465                 470                 475                 480
   gca cag agc ttc tac tgg gtg gta ctg tgt gtg gtg gcc ctg aac aca     1608
   Ala Gln Ser Phe Tyr Trp Val Val Leu Cys Val Val Ala Leu Asn Thr
                   485                 490                 495
   ctg tgt gtg gcc atg gtg cac tat aat cag cct cag cgg ctt acc act     1656
   Leu Cys Val Ala Met Val His Tyr Asn Gln Pro Gln Arg Leu Thr Thr
               500                 505                 510
   gca ctg tac ttt gca gag ttt gtt ttc ctg ggt ctc ttc ctc aca gag     1704
   Ala Leu Tyr Phe Ala Glu Phe Val Phe Leu Gly Leu Phe Leu Thr Glu
   515 520 525
   atg tcc ctg aag atg tat ggc cta ggg ccc aga agt tac ttc agg tct 1752
   Met Ser Leu Lys Met Tyr Gly Leu Gly Pro Arg Ser Tyr Phe Arg Ser
   530 535 540
   tcc ttc aac tgc ttt gac ttt ggg gtg att gtg ggg agt atc ttt gaa 1800
   Ser Phe Asn Cys Phe Asp Phe Gly Val Ile Val Gly Ser Ile Phe Glu
   545 550 555 560
   gta gtc tgg gct gcc atc aag cca gga acc tcc ttt gga atc agt gtg 1848
   Val Val Trp Ala Ala Ile Lys Pro Gly Thr Ser Phe Gly Ile Ser Val
   565 570 575
   ctg cgg gct ctg cga ctg ctg agg ata ttc aaa gtt acc aag tat tgg 1896
   Leu Arg Ala Leu Arg Leu Leu Arg Ile Phe Lys Val Thr Lys Tyr Trp
   580 585 590
   aac tct ctg agg aac ctg gtg gtt tcc ctc ctc aat tcc atg aag tcc 1944
   Asn Ser Leu Arg Asn Leu Val Val Ser Leu Leu Asn Ser Met Lys Ser
   595 600 605
   atc atc agc ctt ctc ttc ctg ctt ttc ctc ttc atc gtg gtc ttc gct 1992
   Ile Ile Ser Leu Leu Phe Leu Leu Phe Leu Phe Ile Val Val Phe Ala
   610 615 620
   ctg ttg ggg atg cag ctg ttc gga gga cag ttc aac ttt caa gat gag 2040
   Leu Leu Gly Met Gln Leu Phe Gly Gly Gln Phe Asn Phe Gln Asp Glu
   625 630 635 640
   act cca acc acc att ttt gat acc ttc cca gct gcc atc ctc act gtc 2088
   Thr Pro Thr Thr Ile Phe Asp Thr Phe Pro Ala Ala Ile Leu Thr Val
   645 650 655
   ttt cag atc ctg aca gga gag gat tgg aat gcc gta atg tat cat ggg 2136
   Phe Gln Ile Leu Thr Gly Glu Asp Trp Asn Ala Val Met Tyr His Gly
   660 665 670
   att gag tca caa ggt gga gtc agc aaa ggc atg ttt tct tcc ttt tac 2184
   Ile Glu Ser Gln Gly Gly Val Ser Lys Gly Met Phe Ser Ser Phe Tyr
   675 680 685
   ttc atc gtc ctg aca ctg ttt gga aac tac acc ctg ctg aat gtt ttt 2232
   Phe Ile Val Leu Thr Leu Phe Gly Asn Tyr Thr Leu Leu Asn Val Phe
   690 695 700
   ctg gcc att gct gtg gac aac ctt gcc aat gcc cag gag ttg acc aag 2280
   Leu Ala Ile Ala Val Asp Asn Leu Ala Asn Ala Gln Glu Leu Thr Lys
   705 710 715 720
   gat gaa gag gag atg gaa gaa gca gcc aat cag aaa ctt gct ctt cag 2328
   Asp Glu Glu Glu Met Glu Glu Ala Ala Asn Gln Lys Leu Ala Leu Gln
   725 730 735
   aag gcc aaa gaa gta gct gaa gtc agc ccc atg tct gct gcc aat atc 2376
   Lys Ala Lys Glu Val Ala Glu Val Ser Pro Met Ser Ala Ala Asn Ile
   740 745 750
   tcc atc gct gcg cag gag aac tcg gcc aag gcg cgc tca gta tgg gag 2424
   Ser Ile Ala Ala Gln Glu Asn Ser Ala Lys Ala Arg Ser Val Trp Glu
   755 760 765
   cag cgg gcc agt cag cta agg ctc cag aat ctg cgt gcc agc tgt gag 2472
   Gln Arg Ala Ser Gln Leu Arg Leu Gln Asn Leu Arg Ala Ser Cys Glu
   770 775 780
   gca ttg tac agt gag atg gac cct gag gag cgc ctg cgt tat gcc agc 2520
   Ala Leu Tyr Ser Glu Met Asp Pro Glu Glu Arg Leu Arg Tyr Ala Ser
   785 790 795 800
   acg cgc cat gtg agg cca gac atg aag aca cac atg gac cga ccc cta 2568
   Thr Arg His Val Arg Pro Asp Met Lys Thr His Met Asp Arg Pro Leu
   805 810 815
   gtg gtg gag cct ggt cga gat ggc ttg cgg gga ccc gtt ggg agc aag 2616
   Val Val Glu Pro Gly Arg Asp Gly Leu Arg Gly Pro Val Gly Ser Lys
   820 825 830
   tca aag cct gaa ggc acg gag gcc aca gaa agc gcg gac cta cct cgc 2664
   Ser Lys Pro Glu Gly Thr Glu Ala Thr Glu Ser Ala Asp Leu Pro Arg
   835 840 845
   cgg cac cac cgg cat cgt gat agg gac aag acc tca gcc aca gca cct 2712
   Arg His His Arg His Arg Asp Arg Asp Lys Thr Ser Ala Thr Ala Pro
   850 855 860
   gct gga ggc gaa cag gac agg aca gaa agc acc gag acc ggg ccc cgg 2760
   Ala Gly Gly Glu Gln Asp Arg Thr Glu Ser Thr Glu Thr Gly Pro Arg
   865 870 875 880
   gag gaa cgt gcg cgc cct cgt cga agt cac agc aag gag act cca ggg 2808
   Glu Glu Arg Ala Arg Pro Arg Arg Ser His Ser Lys Glu Thr Pro Gly
   885 890 895
   gct gac acg caa gtg cgc tgt gag cgc agt agg cgt cac cac cgg cgc 2856
   Ala Asp Thr Gln Val Arg Cys Glu Arg Ser Arg Arg His His Arg Arg
   900 905 910
   ggc tcc ccg gag gag gcc act gaa cgg gag cct cgg cgc cac cgt gcc 2904
   Gly Ser Pro Glu Glu Ala Thr Glu Arg Glu Pro Arg Arg His Arg Ala
   915 920 925
   cac cgg cat gca cag gac tca agc aag gag ggc acg gcg ccg gtg ctt 2952
   His Arg His Ala Gln Asp Ser Ser Lys Glu Gly Thr Ala Pro Val Leu
   930 935 940
   gta ccc aag ggt gag cga cga gca aga cac cga ggc cca cgc acg ggt 3000
   Val Pro Lys Gly Glu Arg Arg Ala Arg His Arg Gly Pro Arg Thr Gly
   945 950 955 960
   cca cgt gag gca gag aac aac gag gag ccc aca cgc agg cac cgt gca 3048
   Pro Arg Glu Ala Glu Asn Asn Glu Glu Pro Thr Arg Arg His Arg Ala
   965 970 975
   agg cat aag gtg cca ccc aca ctg cag ccc cca gag agg gag gct gca 3096
   Arg His Lys Val Pro Pro Thr Leu Gln Pro Pro Glu Arg Glu Ala Ala
   980 985 990
   gag aag gag agc aac gcg gtg gaa ggg gat aag gaa acc cga aat cac 3144
   Glu Lys Glu Ser Asn Ala Val Glu Gly Asp Lys Glu Thr Arg Asn His
   995 1000 1005
   cag ccc aag gaa cct cac tgt gac ctg gag gcc att gca gtt aca ggt 3192
   Gln Pro Lys Glu Pro His Cys Asp Leu Glu Ala Ile Ala Val Thr Gly
   1010 1015 1020
   gtg ggc cct ctg cac atg ctg ccc agc acc tgt ctc cag aaa gtg gac 3240
   Val Gly Pro Leu His Met Leu Pro Ser Thr Cys Leu Gln Lys Val Asp
   1025 1030 1035 1040
   gag caa cca gag gat gca gac aac cag cgt aat gtc acc cgg atg ggc 3288
   Glu Gln Pro Glu Asp Ala Asp Asn Gln Arg Asn Val Thr Arg Met Gly
   1045 1050 1055
   agt cag ccc tca gat ccc agc acc act gtg cat gtc cca gtg aca ctg 3336
   Ser Gln Pro Ser Asp Pro Ser Thr Thr Val His Val Pro Val Thr Leu
   1060 1065 1070
   aca ggc cct cct ggg gag acc cct gta gtt ccc agt ggt aac atg aac 3384
   Thr Gly Pro Pro Gly Glu Thr Pro Val Val Pro Ser Gly Asn Met Asn
   1075 1080 1085
   ctg gaa ggc caa gca gag ggc aag aag gag gca gag gcg gat gat gtg 3432
   Leu Glu Gly Gln Ala Glu Gly Lys Lys Glu Ala Glu Ala Asp Asp Val
   1090 1095 1100
   ctg aga aga ggc ccc agg ccc atc gtt ccc tac agc tcc atg ttt tgt 3480
   Leu Arg Arg Gly Pro Arg Pro Ile Val Pro Tyr Ser Ser Met Phe Cys
   1105 1110 1115 1120
   ctc agc ccc acc aac ctg ttt cgt cgc ttc tgc cat tac att gtg acc 3528
   Leu Ser Pro Thr Asn Leu Phe Arg Arg Phe Cys His Tyr Ile Val Thr
   1125 1130 1135
   atg cgg tac ttg gag atg gta att ctt gtg gtc att gcc ttg agc agc 3576
   Met Arg Tyr Leu Glu Met Val Ile Leu Val Val Ile Ala Leu Ser Ser
   1140 1145 1150
   att gcc ctg gct gca gag gat cct gtg cgg aca gat tca ttc agg aac 3624
   Ile Ala Leu Ala Ala Glu Asp Pro Val Arg Thr Asp Ser Phe Arg Asn
   1155 1160 1165
   aac gct tta gag tac atg gat tac atc ttt aca gga gtc ttc acc tgt 3672
   Asn Ala Leu Glu Tyr Met Asp Tyr Ile Phe Thr Gly Val Phe Thr Cys
   1170 1175 1180
   gaa atg gtc ata aag atg ata gac ttg ggc ttg ctg ctg cac cct ggt 3720
   Glu Met Val Ile Lys Met Ile Asp Leu Gly Leu Leu Leu His Pro Gly
   1185 1190 1195 1200
   gcc tac ttc cgg gac ctg tgg aac att ctg gac ttc atc gtt gtc agt 3768
   Ala Tyr Phe Arg Asp Leu Trp Asn Ile Leu Asp Phe Ile Val Val Ser
   1205 1210 1215
   gga gcc ctg gtg gca ttt gcg ttc tca gga tcc aaa ggg aaa gac atc 3816
   Gly Ala Leu Val Ala Phe Ala Phe Ser Gly Ser Lys Gly Lys Asp Ile
   1220 1225 1230
   aat acc atc aag tct ctg aga gtc ctg cgt gtc ctg agg ccc ctc aag 3864
   Asn Thr Ile Lys Ser Leu Arg Val Leu Arg Val Leu Arg Pro Leu Lys
   1235 1240 1245
   acc atc aag cgg ctg cct aaa ctc aag gct gtc ttt gac tgt gtg gtg 3912
   Thr Ile Lys Arg Leu Pro Lys Leu Lys Ala Val Phe Asp Cys Val Val
   1250 1255 1260
   aac tcc ctg aag aac gtc ttg aac atc ctg att gtc tac atg ctc ttc 3960
   Asn Ser Leu Lys Asn Val Leu Asn Ile Leu Ile Val Tyr Met Leu Phe
   1265 1270 1275 1280
   atg ttc ata ttt gcc gtc att gcc gtc cag ctc ttc aaa ggg aag ttc 4008
   Met Phe Ile Phe Ala Val Ile Ala Val Gln Leu Phe Lys Gly Lys Phe
   1285 1290 1295
   ttt tac tgt act gat gaa tcc aag gag ctg gag agg gac tgc cgg ggt 4056
   Phe Tyr Cys Thr Asp Glu Ser Lys Glu Leu Glu Arg Asp Cys Arg Gly
   1300 1305 1310
   cag tat ttg gat tat gag aag gaa gaa gta gaa gcc cag cca agg cag 4104
   Gln Tyr Leu Asp Tyr Glu Lys Glu Glu Val Glu Ala Gln Pro Arg Gln
   1315 1320 1325
   tgg aag aaa tat gac ttc cac tat gac aat gtt ctc tgg gcc ttg ttg 4152
   Trp Lys Lys Tyr Asp Phe His Tyr Asp Asn Val Leu Trp Ala Leu Leu
   1330 1335 1340
   acg ctg ttc aca gtg tcc acg gga gag ggg tgg ccc atg gtg ctg aaa 4200
   Thr Leu Phe Thr Val Ser Thr Gly Glu Gly Trp Pro Met Val Leu Lys
   1345 1350 1355 1360
   cac tct gtg gat gcc acc tat gag gaa cag ggg ccc agt ccc ggc ttc 4248
   His Ser Val Asp Ala Thr Tyr Glu Glu Gln Gly Pro Ser Pro Gly Phe
   1365 1370 1375
   cgg atg gag ctc tcc atc ctc tac gtg gtc tac ttt gtg gtc ttc cct 4296
   Arg Met Glu Leu Ser Ile Leu Tyr Val Val Tyr Phe Val Val Phe Pro
   1380 1385 1390
   ttt ttc ttt gtc aac atc ttt gtg gcc ttg atc att atc acc ttc cag 4344
   Phe Phe Phe Val Asn Ile Phe Val Ala Leu Ile Ile Ile Thr Phe Gln
   1395 1400 1405
   gaa cag gga gat aag gtg atg tct gaa tgc agc tta gaa aag aat gag 4392
   Glu Gln Gly Asp Lys Val Met Ser Glu Cys Ser Leu Glu Lys Asn Glu
   1410 1415 1420
   agg gct tgc att gat ttt gcc atc agt gcc aag ccc ctg aca cgg tac 4440
   Arg Ala Cys Ile Asp Phe Ala Ile Ser Ala Lys Pro Leu Thr Arg Tyr
   1425 1430 1435 1440
   atg cct caa aac aaa cag tcg ttc cag tat aag aca tgg aca ttc gtg 4488
   Met Pro Gln Asn Lys Gln Ser Phe Gln Tyr Lys Thr Trp Thr Phe Val
   1445 1450 1455
   gtc tct cca ccc ttt gag tac ttc atc atg gct atg ata gcc ctc aac 4536
   Val Ser Pro Pro Phe Glu Tyr Phe Ile Met Ala Met Ile Ala Leu Asn
   1460 1465 1470
   aca gtg gtg ctg atg atg aag ttc tat gat gca cct tat gag tac gag 4584
   Thr Val Val Leu Met Met Lys Phe Tyr Asp Ala Pro Tyr Glu Tyr Glu
   1475 1480 1485
   ctg atg ctg aaa tgc ctg aac att gtc ttc aca tcc atg ttc tcg atg 4632
   Leu Met Leu Lys Cys Leu Asn Ile Val Phe Thr Ser Met Phe Ser Met
   1490 1495 1500
   gag tgc ata ctg aag atc atc gcc ttt ggg gta ttg aac tac ttc aga 4680
   Glu Cys Ile Leu Lys Ile Ile Ala Phe Gly Val Leu Asn Tyr Phe Arg
   1505 1510 1515 1520
   gat gcc tgg aat gtc ttt gac ttt gtc acg gtt ttg gga agt att act 4728
   Asp Ala Trp Asn Val Phe Asp Phe Val Thr Val Leu Gly Ser Ile Thr
   1525 1530 1535
   gat att tta gta aca gag att gcg gaa acg aac aac ttc atc aac cta 4776
   Asp Ile Leu Val Thr Glu Ile Ala Glu Thr Asn Asn Phe Ile Asn Leu
   1540 1545 1550
   agc ttc ctt cgc ctc ttc cgg gcg gca cgg ctg atc aag ctg ctt cgc 4824
   Ser Phe Leu Arg Leu Phe Arg Ala Ala Arg Leu Ile Lys Leu Leu Arg
   1555 1560 1565
   cag ggc tac acc atc cgc atc cta ttg tgg acc ttc gtc cag tcc ttt 4872
   Gln Gly Tyr Thr Ile Arg Ile Leu Leu Trp Thr Phe Val Gln Ser Phe
   1570 1575 1580
   aag gcg ctg ccc tac gtg tgc ctc ctc att gcc atg ctg ttc ttc atc 4920
   Lys Ala Leu Pro Tyr Val Cys Leu Leu Ile Ala Met Leu Phe Phe Ile
   1585 1590 1595 1600
   tac gcc atc atc gga atg cag gtt ttt gga aac agt gcc ctt gat gat 4968
   Tyr Ala Ile Ile Gly Met Gln Val Phe Gly Asn Ser Ala Leu Asp Asp
   1605 1610 1615
   gac acc agt atc aac cga cac aac aac ttc cgg aca ttt ctg caa gcc 5016
   Asp Thr Ser Ile Asn Arg His Asn Asn Phe Arg Thr Phe Leu Gln Ala
   1620 1625 1630
   ata atg cta ttg ttc agg agt gcc act ggg gag gcc tgg cat gag atc 5064
   Ile Met Leu Leu Phe Arg Ser Ala Thr Gly Glu Ala Trp His Glu Ile
   1635 1640 1645
   atg ctg tca tgt ctg gac aac cgg gcc tgt gac cca cat gcc aac gcc 5112
   Met Leu Ser Cys Leu Asp Asn Arg Ala Cys Asp Pro His Ala Asn Ala
   1650 1655 1660
   agt gag tgc ggg agc gac ttt gcc tat ttt tat ttt gtc tcc ttc atc 5160
   Ser Glu Cys Gly Ser Asp Phe Ala Tyr Phe Tyr Phe Val Ser Phe Ile
   1665 1670 1675 1680
   ttc ctc tgt tcc ttt ctg atg ttg aac ctc ttt gtt gct gta atc atg 5208
   Phe Leu Cys Ser Phe Leu Met Leu Asn Leu Phe Val Ala Val Ile Met
   1685 1690 1695
   gac aat ttt gag tac ctc act cgg gac tct tcc atc cta ggg cct cac 5256
   Asp Asn Phe Glu Tyr Leu Thr Arg Asp Ser Ser Ile Leu Gly Pro His
   1700 1705 1710
   cac tta gac gaa ttc att cga gtc tgg gct gaa tac gac cca gct gcg 5304
   His Leu Asp Glu Phe Ile Arg Val Trp Ala Glu Tyr Asp Pro Ala Ala
   1715 1720 1725
   tgt ggg cgc atc agt tac aat gac atg ttt gag atg ctg aaa cac atg 5352
   Cys Gly Arg Ile Ser Tyr Asn Asp Met Phe Glu Met Leu Lys His Met
   1730 1735 1740
   tcc cca cct ctg ggg ttg ggg aag aaa tgc ccg gct cga gtt gca tac 5400
   Ser Pro Pro Leu Gly Leu Gly Lys Lys Cys Pro Ala Arg Val Ala Tyr
   1745 1750 1755 1760
   aag cgc ctg gtt cgc atg aac atg ccc ata tcc aat gag gac atg acg 5448
   Lys Arg Leu Val Arg Met Asn Met Pro Ile Ser Asn Glu Asp Met Thr
   1765 1770 1775
   gtg cac ttt acg tcc aca ctg atg gcc ctc atc cgg aca gca ctg gag 5496
   Val His Phe Thr Ser Thr Leu Met Ala Leu Ile Arg Thr Ala Leu Glu
   1780 1785 1790
   atc aag ctt gcc cca gct gac gag atg aca gtg ggg aag gtc tat gct 5544
   Ile Lys Leu Ala Pro Ala Asp Glu Met Thr Val Gly Lys Val Tyr Ala
   1795 1800 1805
   gct ctc atg ata ttt gac ttc tac aaa cag aac aaa acc acc aga gat 5592
   Ala Leu Met Ile Phe Asp Phe Tyr Lys Gln Asn Lys Thr Thr Arg Asp
   1810 1815 1820
   cag act cac caa gct ccc gga ggc ctg tcc cag atg ggt ccc gtt tcc 5640
   Gln Thr His Gln Ala Pro Gly Gly Leu Ser Gln Met Gly Pro Val Ser
   1825 1830 1835 1840
   ctg ttc cac cct ctg aag gcc acc ctg gaa cag aca cag ccc gct gtg 5688
   Leu Phe His Pro Leu Lys Ala Thr Leu Glu Gln Thr Gln Pro Ala Val
   1845 1850 1855
   ctt cga gga gct cgg gtt ttc ctt cgg caa aag agt gca act tcc ctc 5736
   Leu Arg Gly Ala Arg Val Phe Leu Arg Gln Lys Ser Ala Thr Ser Leu
   1860 1865 1870
   agc aat ggg ggt gcc ata caa acc cag gaa agt gga tca agg agt cgc 5784
   Ser Asn Gly Gly Ala Ile Gln Thr Gln Glu Ser Gly Ser Arg Ser Arg
   1875 1880 1885
   tgt cct ggg gga cgc aga ggg acc caa gat gca ctt tat gag ggc aga 5832
   Cys Pro Gly Gly Arg Arg Gly Thr Gln Asp Ala Leu Tyr Glu Gly Arg
       1890                1895                1900
   gca cct cta gaa cgt gac cat tct aaa gag atc cct gtg ggg cag tca    5880
   Ala Pro Leu Glu Arg Asp His Ser Lys Glu Ile Pro Val Gly Gln Ser
   1905                1910                1915                1920
   gga aca ctg ctg gtg gat gtc cag atg cag aac atg aca ctg aga gga    5928
   Gly Thr Leu Leu Val Asp Val Gln Met Gln Asn Met Thr Leu Arg Gly
                   1925                1930                1935
   cca gat ggg gat ccc cag cct ggc ctg gaa agc caa ggc aga gct gcc    5976
   Pro Asp Gly Asp Pro Gln Pro Gly Leu Glu Ser Gln Gly Arg Ala Ala
               1940                1945                1950
   tct atg cta cgc cta gcg gca gaa aca cag ccg gcc cct aat gcc agc    6024
   Ser Met Leu Arg Leu Ala Ala Glu Thr Gln Pro Ala Pro Asn Ala Ser
           1955                1960                1965
   ccc atg aag cgc tcc atc tcc aca ctg gct cca cgc cca gat ggg act    6072
   Pro Met Lys Arg Ser Ile Ser Thr Leu Ala Pro Arg Pro Asp Gly Thr
       1970                1975                1980
   cag ctt tgc agc aca gtt ctg gac cgg cct cct cct agc cag gca tca    6120
   Gln Leu Cys Ser Thr Val Leu Asp Arg Pro Pro Pro Ser Gln Ala Ser
   1985                1990                1995                2000
   cat cac cac cac cac cgc tgc cac cgg cgc aga gac aag aag caa agg    6168
   His His His His His Arg Cys His Arg Arg Arg Asp Lys Lys Gln Arg
                   2005                2010                2015
   tcc ctg gaa aag ggg ccc agc ctg tct gtt gac cca gaa ggt gca cca    6216
   Ser Leu Glu Lys Gly Pro Ser Leu Ser Val Asp Pro Glu Gly Ala Pro
               2020                2025                2030
   agc act gct gct gca gga cct ggt ctg ccc cat gga gaa gga tcc acc    6264
   Ser Thr Ala Ala Ala Gly Pro Gly Leu Pro His Gly Glu Gly Ser Thr
           2035                2040                2045
   gcc tgc cgg cgg gac cgt aaa cag gag cga ggc cgg tcc cag gag cgg    6312
   Ala Cys Arg Arg Asp Arg Lys Gln Glu Arg Gly Arg Ser Gln Glu Arg
       2050                2055                2060
   agg cag ccc tca tct tcc tct tca gag aag cag cgc ttc tat tcc tgt    6360
   Arg Gln Pro Ser Ser Ser Ser Ser Glu Lys Gln Arg Phe Tyr Ser Cys
   2065                2070                2075                2080
   gac cgc ttg gga gcc ggg agc ccc caa ctg atg ccc tca ctc agt agc    6408
   Asp Arg Leu Gly Ala Gly Ser Pro Gln Leu Met Pro Ser Leu Ser Ser
                   2085                2090                2095
   cac ccc aca tcg ccg gcg gcg gcg cta gag cca gca ccc cac cca cag    6456
   His Pro Thr Ser Pro Ala Ala Ala Leu Glu Pro Ala Pro His Pro Gln
               2100                2105                2110
   ggc agt ggt tcc gtt aat ggg agc ccc ttg atg tca aca tcc ggt gct    6504
   Gly Ser Gly Ser Val Asn Gly Ser Pro Leu Met Ser Thr Ser Gly Ala
           2115                2120                2125
   att act ccc ggg cga ggt ggg cgg agg cag ctc ccc cag act cct ctg    6552
   Ile Thr Pro Gly Arg Gly Gly Arg Arg Gln Leu Pro Gln Thr Pro Leu
       2130                2135                2140
   acc cca cgc ccc agc atc acc tac aag acc gcc aat tcc tcg cct gtc    6600
   Thr Pro Arg Pro Ser Ile Thr Tyr Lys Thr Ala Asn Ser Ser Pro Val
   2145                2150                2155                2160
   cac ttt gct gag ggt cag agc ggc ctc cca gcc ttc tcc cct ggc cgt    6648
   His Phe Ala Glu Gly Gln Ser Gly Leu Pro Ala Phe Ser Pro Gly Arg
                   2165                2170                2175
   ctc agc cgc ggc ctt tct gaa cac aat gcc ctg ctc cag aaa gag ccc    6696
   Leu Ser Arg Gly Leu Ser Glu His Asn Ala Leu Leu Gln Lys Glu Pro
               2180                2185                2190
   ctg agc cag cct cta gct cct ggc tcc cga att ggc tct gac cct tac    6744
   Leu Ser Gln Pro Leu Ala Pro Gly Ser Arg Ile Gly Ser Asp Pro Tyr
           2195                2200                2205
   cta ggg cag cgt ctg gac agt gag gcc tcc gcc cac acc ctg cct gag    6792
   Leu Gly Gln Arg Leu Asp Ser Glu Ala Ser Ala His Thr Leu Pro Glu
       2210                2215                2220
   gat aca ctc acc ttt gaa gag gca gtg gcc acc aac tct ggc cgc tcc    6840
   Asp Thr Leu Thr Phe Glu Glu Ala Val Ala Thr Asn Ser Gly Arg Ser
   2225                2230                2235                2240
   tcc agg act tcc tat gtg tcc tcc ctc act tcc caa tcc cac cct ctc    6888
   Ser Arg Thr Ser Tyr Val Ser Ser Leu Thr Ser Gln Ser His Pro Leu
                   2245                2250                2255
   cgc cgt gta ccc aat ggc tat cac tgc act ttg gga ctc aac act ggc    6936
   Arg Arg Val Pro Asn Gly Tyr His Cys Thr Leu Gly Leu Asn Thr Gly
               2260                2265                2270
   gtc ggg gca cga gca agc tac cac cac ccc gat cag gac cac tgg tgc    6984
   Val Gly Ala Arg Ala Ser Tyr His His Pro Asp Gln Asp His Trp Cys
           2275                2280                2285
   tagcttcacc acgaccaccc atgtaccagc tccatgggtg agggttccag ttgatgagtt 7044
   ttatcatccc actctggact gtggggtcac aaccctggga ggagggccct cacatctcgg 7104
   cctctgtggt ggaggctcct gcttccctcc ctccctccct ttttacactg gatagactaa 7164
   taaagccctt tcttagaggg g                                           7185
<210> 2
   <211> 2288
   <212> PRT
   <213> Mus musculus
<400> 2
   Met Val Arg Phe Gly Asp Glu Leu Gly Gly Arg Tyr Gly Gly Thr Gly
   1               5                   10                  15
   Gly Gly Glu Arg Ala Arg Gly Gly Gly Ala Gly Gly Ala Gly Gly Pro
   20 25 30
   Gly Gln Gly Gly Leu Pro Pro Gly Gln Arg Val Leu Tyr Lys Gln Ser
   35 40 45
   Ile Ala Gln Arg Ala Arg Thr Met Ala Leu Tyr Asn Pro Ile Pro Val
   50 55 60
   Lys Gln Asn Cys Phe Thr Val Asn Arg Ser Leu Phe Val Phe Ser Glu
   65 70 75 80
   Asp Asn Val Val Arg Lys Tyr Ala Lys Arg Ile Thr Glu Trp Pro Pro
   85 90 95
   Phe Glu Tyr Met Ile Leu Ala Thr Ile Ile Ala Asn Cys Ile Val Leu
   100 105 110
   Ala Leu Glu Gln His Leu Pro Asp Gly Asp Lys Thr Pro Met Ser Glu
   115 120 125
   Arg Leu Asp Asp Thr Glu Pro Tyr Phe Ile Gly Ile Phe Cys Phe Glu
   130 135 140
   Ala Gly Ile Lys Ile Ile Ala Leu Gly Phe Val Phe His Lys Gly Ser
   145 150 155 160
   Tyr Leu Arg Asn Gly Trp Asn Val Met Asp Phe Val Val Val Leu Thr
   165 170 175
   Gly Ile Leu Ala Thr Ala Gly Thr Asp Phe Asp Leu Arg Thr Leu Arg
   180 185 190
   Ala Val Arg Val Leu Arg Pro Leu Lys Leu Val Ser Gly Ile Pro Ser
   195 200 205
   Leu Gln Val Val Leu Lys Ser Ile Met Lys Ala Met Val Pro Leu Leu
   210 215 220
   Gln Ile Gly Leu Leu Leu Phe Phe Ala Ile Leu Met Phe Gly Ile Ile
   225 230 235 240
   Gly Leu Glu Phe Tyr Met Gly Lys Phe His Lys Ala Cys Phe Pro Asn
   245 250 255
   Ser Thr Asp Thr Glu Pro Val Gly Asp Phe Pro Cys Gly Lys Asp Pro
   260 265 270
   Pro Ala Arg Gln Cys Asp Gly Asp Thr Glu Cys Arg Glu Tyr Trp Pro
   275 280 285
   Gly Pro Asn Phe Gly Ile Thr Asn Phe Asp Asn Ile Leu Phe Ala Ile
   290 295 300
   Leu Thr Val Phe Gln Cys Ile Thr Met Glu Gly Trp Thr Asp Ile Leu
   305 310 315 320
   Tyr Asn Thr Asn Asp Ala Ala Gly Asn Thr Trp Asn Trp Leu Tyr Phe
   325 330 335
   Ile Pro Leu Ile Ile Ile Gly Ser Phe Phe Met Leu Asn Leu Val Leu
   340 345 350
   Gly Val Leu Ser Gly Glu Phe Ala Lys Glu Arg Glu Arg Val Glu Asn
   355 360 365
   Arg Arg Ala Phe Leu Lys Leu Arg Arg Gln Gln Gln Ile Glu Arg Glu
   370 375 380
   Leu Asn Gly Tyr Leu Glu Trp Ile Phe Lys Ala Glu Glu Val Met Leu
   385 390 395 400
   Ala Glu Glu Asp Lys Asn Ala Glu Glu Lys Ser Pro Leu Asp Val Leu
   405 410 415
   Lys Arg Ala Ala Thr Lys Lys Ser Arg Asn Asp Leu Ile His Ala Glu
   420 425 430
   Glu Gly Glu Asp Arg Phe Val Asp Leu Cys Ala Val Gly Ser Pro Phe
   435 440 445
   Ala Arg Ala Ser Leu Lys Ser Gly Lys Thr Glu Ser Ser Ser Tyr Phe
   450 455 460
   Arg Arg Lys Glu Lys Met Phe Arg Phe Phe Ile Arg Arg Met Val Lys
   465 470 475 480
   Ala Gln Ser Phe Tyr Trp Val Val Leu Cys Val Val Ala Leu Asn Thr
   485 490 495
   Leu Cys Val Ala Met Val His Tyr Asn Gln Pro Gln Arg Leu Thr Thr
   500 505 510
   Ala Leu Tyr Phe Ala Glu Phe Val Phe Leu Gly Leu Phe Leu Thr Glu
   515 520 525
   Met Ser Leu Lys Met Tyr Gly Leu Gly Pro Arg Ser Tyr Phe Arg Ser
   530 535 540
   Ser Phe Asn Cys Phe Asp Phe Gly Val Ile Val Gly Ser Ile Phe Glu
   545 550 555 560
   Val Val Trp Ala Ala Ile Lys Pro Gly Thr Ser Phe Gly Ile Ser Val
   565 570 575
   Leu Arg Ala Leu Arg Leu Leu Arg Ile Phe Lys Val Thr Lys Tyr Trp
   580 585 590
   Asn Ser Leu Arg Asn Leu Val Val Ser Leu Leu Asn Ser Met Lys Ser
   595 600 605
   Ile Ile Ser Leu Leu Phe Leu Leu Phe Leu Phe Ile Val Val Phe Ala
   610 615 620
   Leu Leu Gly Met Gln Leu Phe Gly Gly Gln Phe Asn Phe Gln Asp Glu
   625 630 635 640
   Thr Pro Thr Thr Ile Phe Asp Thr Phe Pro Ala Ala Ile Leu Thr Val
   645 650 655
   Phe Gln Ile Leu Thr Gly Glu Asp Trp Asn Ala Val Met Tyr His Gly
   660 665 670
   Ile Glu Ser Gln Gly Gly Val Ser Lys Gly Met Phe Ser Ser Phe Tyr
   675 680 685
   Phe Ile Val Leu Thr Leu Phe Gly Asn Tyr Thr Leu Leu Asn Val Phe
   690 695 700
   Leu Ala Ile Ala Val Asp Asn Leu Ala Asn Ala Gln Glu Leu Thr Lys
   705 710 715 720
   Asp Glu Glu Glu Met Glu Glu Ala Ala Asn Gln Lys Leu Ala Leu Gln
   725 730 735
   Lys Ala Lys Glu Val Ala Glu Val Ser Pro Met Ser Ala Ala Asn Ile
   740 745 750
   Ser Ile Ala Ala Gln Glu Asn Ser Ala Lys Ala Arg Ser Val Trp Glu
   755 760 765
   Gln Arg Ala Ser Gln Leu Arg Leu Gln Asn Leu Arg Ala Ser Cys Glu
   770 775 780
   Ala Leu Tyr Ser Glu Met Asp Pro Glu Glu Arg Leu Arg Tyr Ala Ser
   785 790 795 800
   Thr Arg His Val Arg Pro Asp Met Lys Thr His Met Asp Arg Pro Leu
   805 810 815
   Val Val Glu Pro Gly Arg Asp Gly Leu Arg Gly Pro Val Gly Ser Lys
   820 825 830
   Ser Lys Pro Glu Gly Thr Glu Ala Thr Glu Ser Ala Asp Leu Pro Arg
   835 840 845
   Arg His His Arg His Arg Asp Arg Asp Lys Thr Ser Ala Thr Ala Pro
   850 855 860
   Ala Gly Gly Glu Gln Asp Arg Thr Glu Ser Thr Glu Thr Gly Pro Arg
   865 870 875 880
   Glu Glu Arg Ala Arg Pro Arg Arg Ser His Ser Lys Glu Thr Pro Gly
   885 890 895
   Ala Asp Thr Gln Val Arg Cys Glu Arg Ser Arg Arg His His Arg Arg
   900 905 910
   Gly Ser Pro Glu Glu Ala Thr Glu Arg Glu Pro Arg Arg His Arg Ala
   915 920 925
   His Arg His Ala Gln Asp Ser Ser Lys Glu Gly Thr Ala Pro Val Leu
   930 935 940
   Val Pro Lys Gly Glu Arg Arg Ala Arg His Arg Gly Pro Arg Thr Gly
   945 950 955 960
   Pro Arg Glu Ala Glu Asn Asn Glu Glu Pro Thr Arg Arg His Arg Ala
   965 970 975
   Arg His Lys Val Pro Pro Thr Leu Gln Pro Pro Glu Arg Glu Ala Ala
   980 985 990
   Glu Lys Glu Ser Asn Ala Val Glu Gly Asp Lys Glu Thr Arg Asn His
   995 1000 1005
   Gln Pro Lys Glu Pro His Cys Asp Leu Glu Ala Ile Ala Val Thr Gly
   1010 1015 1020
   Val Gly Pro Leu His Met Leu Pro Ser Thr Cys Leu Gln Lys Val Asp
   1025 1030 1035 1040
   Glu Gln Pro Glu Asp Ala Asp Asn Gln Arg Asn Val Thr Arg Met Gly
               1045                1050                1055
   Ser Gln Pro Ser Asp Pro Ser Thr Thr Val His Val Pro Val Thr Leu
           1060                1065                1070
   Thr Gly Pro Pro Gly Glu Thr Pro Val Val Pro Ser Gly Asn Met Asn
       1075                1080                1085
   Leu Glu Gly Gln Ala Glu Gly Lys Lys Glu Ala Glu Ala Asp Asp Val
   1090                1095                1100
   Leu Arg Arg Gly Pro Arg Pro Ile Val Pro Tyr Ser Ser Met Phe Cys
   1105                1110                1115                1120
   Leu Ser Pro Thr Asn Leu Phe Arg Arg Phe Cys His Tyr Ile Val Thr
                   1125                1130                1135
   Met Arg Tyr Leu Glu Met Val Ile Leu Val Val Ile Ala Leu Ser Ser
               1140                1145                1150
   Ile Ala Leu Ala Ala Glu Asp Pro Val Arg Thr Asp Ser Phe Arg Asn
           1155                1160                1165
   Asn Ala Leu Glu Tyr Met Asp Tyr Ile Phe Thr Gly Val Phe Thr Cys
       1170                1175                1180
   Glu Met Val Ile Lys Met Ile Asp Leu Gly Leu Leu Leu His Pro Gly
   1185                1190                1195                1200
   Ala Tyr Phe Arg Asp Leu Trp Asn Ile Leu Asp Phe Ile Val Val Ser
                   1205                1210                1215
   Gly Ala Leu Val Ala Phe Ala Phe Ser Gly Ser Lys Gly Lys Asp Ile
               1220                1225                1230
   Asn Thr Ile Lys Ser Leu Arg Val Leu Arg Val Leu Arg Pro Leu Lys
           1235                1240                1245
   Thr Ile Lys Arg Leu Pro Lys Leu Lys Ala Val Phe Asp Cys Val Val
       1250                1255                1260
   Asn Ser Leu Lys Asn Val Leu Asn Ile Leu Ile Val Tyr Met Leu Phe
   1265                1270                1275                1280
   Met Phe Ile Phe Ala Val Ile Ala Val Gln Leu Phe Lys Gly Lys Phe
                   1285                1290                1295
   Phe Tyr Cys Thr Asp Glu Ser Lys Glu Leu Glu Arg Asp Cys Arg Gly
               1300                1305                1310
   Gln Tyr Leu Asp Tyr Glu Lys Glu Glu Val Glu Ala Gln Pro Arg Gln
           1315                1320                1325
   Trp Lys Lys Tyr Asp Phe His Tyr Asp Asn Val Leu Trp Ala Leu Leu
       1330                1335                1340
   Thr Leu Phe Thr Val Ser Thr Gly Glu Gly Trp Pro Met Val Leu Lys
   1345                1350                1355                1360
   His Ser Val Asp Ala Thr Tyr Glu Glu Gln Gly Pro Ser Pro Gly Phe
                   1365                1370                1375
   Arg Met Glu Leu Ser Ile Leu Tyr Val Val Tyr Phe Val Val Phe Pro
               1380                1385                1390
   Phe Phe Phe Val Asn Ile Phe Val Ala Leu Ile Ile Ile Thr Phe Gln
   1395 1400 1405
   Glu Gln Gly Asp Lys Val Met Ser Glu Cys Ser Leu Glu Lys Asn Glu
   1410 1415 1420
   Arg Ala Cys Ile Asp Phe Ala Ile Ser Ala Lys Pro Leu Thr Arg Tyr
   1425 1430 1435 1440
   Met Pro Gln Asn Lys Gln Ser Phe Gln Tyr Lys Thr Trp Thr Phe Val
   1445 1450 1455
   Val Ser Pro Pro Phe Glu Tyr Phe Ile Met Ala Met Ile Ala Leu Asn
   1460 1465 1470
   Thr Val Val Leu Met Met Lys Phe Tyr Asp Ala Pro Tyr Glu Tyr Glu
   1475 1480 1485
   Leu Met Leu Lys Cys Leu Asn Ile Val Phe Thr Ser Met Phe Ser Met
   1490 1495 1500
   Glu Cys Ile Leu Lys Ile Ile Ala Phe Gly Val Leu Asn Tyr Phe Arg
   1505 1510 1515 1520
   Asp Ala Trp Asn Val Phe Asp Phe Val Thr Val Leu Gly Ser Ile Thr
   1525 1530 1535
   Asp Ile Leu Val Thr Glu Ile Ala Glu Thr Asn Asn Phe Ile Asn Leu
   1540 1545 1550
   Ser Phe Leu Arg Leu Phe Arg Ala Ala Arg Leu Ile Lys Leu Leu Arg
   1555 1560 1565
   Gln Gly Tyr Thr Ile Arg Ile Leu Leu Trp Thr Phe Val Gln Ser Phe
   1570 1575 1580
   Lys Ala Leu Pro Tyr Val Cys Leu Leu Ile Ala Met Leu Phe Phe Ile
   1585 1590 1595 1600
   Tyr Ala Ile Ile Gly Met Gln Val Phe Gly Asn Ser Ala Leu Asp Asp
   1605 1610 1615
   Asp Thr Ser Ile Asn Arg His Asn Asn Phe Arg Thr Phe Leu Gln Ala
   1620 1625 1630
   Ile Met Leu Leu Phe Arg Ser Ala Thr Gly Glu Ala Trp His Glu Ile
   1635 1640 1645
   Met Leu Ser Cys Leu Asp Asn Arg Ala Cys Asp Pro His Ala Asn Ala
   1650 1655 1660
   Ser Glu Cys Gly Ser Asp Phe Ala Tyr Phe Tyr Phe Val Ser Phe Ile
   1665 1670 1675 1680
   Phe Leu Cys Ser Phe Leu Met Leu Asn Leu Phe Val Ala Val Ile Met
   1685 1690 1695
   Asp Asn Phe Glu Tyr Leu Thr Arg Asp Ser Ser Ile Leu Gly Pro His
   1700 1705 1710
   His Leu Asp Glu Phe Ile Arg Val Trp Ala Glu Tyr Asp Pro Ala Ala
   1715 1720 1725
   Cys Gly Arg Ile Ser Tyr Asn Asp Met Phe Glu Met Leu Lys His Met
   1730 1735 1740
   Ser Pro Pro Leu Gly Leu Gly Lys Lys Cys Pro Ala Arg Val Ala Tyr
   1745 1750 1755 1760
   Lys Arg Leu Val Arg Met Asn Met Pro Ile Ser Asn Glu Asp Met Thr
   1765 1770 1775
   Val His Phe Thr Ser Thr Leu Met Ala Leu Ile Arg Thr Ala Leu Glu
   1780 1785 1790
   Ile Lys Leu Ala Pro Ala Asp Glu Met Thr Val Gly Lys Val Tyr Ala
   1795 1800 1805
   Ala Leu Met Ile Phe Asp Phe Tyr Lys Gln Asn Lys Thr Thr Arg Asp
   1810 1815 1820
   Gln Thr His Gln Ala Pro Gly Gly Leu Ser Gln Met Gly Pro Val Ser
   1825 1830 1835 1840
   Leu Phe His Pro Leu Lys Ala Thr Leu Glu Gln Thr Gln Pro Ala Val
   1845 1850 1855
   Leu Arg Gly Ala Arg Val Phe Leu Arg Gln Lys Ser Ala Thr Ser Leu
   1860 1865 1870
   Ser Asn Gly Gly Ala Ile Gln Thr Gln Glu Ser Gly Ser Arg Ser Arg
   1875 1880 1885
   Cys Pro Gly Gly Arg Arg Gly Thr Gln Asp Ala Leu Tyr Glu Gly Arg
   1890 1895 1900
   Ala Pro Leu Glu Arg Asp His Ser Lys Glu Ile Pro Val Gly Gln Ser
   1905 1910 1915 1920
   Gly Thr Leu Leu Val Asp Val Gln Met Gln Asn Met Thr Leu Arg Gly
   1925 1930 1935
   Pro Asp Gly Asp Pro Gln Pro Gly Leu Glu Ser Gln Gly Arg Ala Ala
   1940 1945 1950
   Ser Met Leu Arg Leu Ala Ala Glu Thr Gln Pro Ala Pro Asn Ala Ser
   1955 1960 1965
   Pro Met Lys Arg Ser Ile Ser Thr Leu Ala Pro Arg Pro Asp Gly Thr
   1970 1975 1980
   Gln Leu Cys Ser Thr Val Leu Asp Arg Pro Pro Pro Ser Gln Ala Ser
   1985 1990 1995 2000
   His His His His His Arg Cys His Arg Arg Arg Asp Lys Lys Gln Arg
   2005 2010 2015
   Ser Leu Glu Lys Gly Pro Ser Leu Ser Val Asp Pro Glu Gly Ala Pro
   2020 2025 2030
   Ser Thr Ala Ala Ala Gly Pro Gly Leu Pro His Gly Glu Gly Ser Thr
   2035 2040 2045
   Ala Cys Arg Arg Asp Arg Lys Gln Glu Arg Gly Arg Ser Gln Glu Arg
   2050 2055 2060
   Arg Gln Pro Ser Ser Ser Ser Ser Glu Lys Gln Arg Phe Tyr Ser Cys
   2065 2070 2075 2080
   Asp Arg Leu Gly Ala Gly Ser Pro Gln Leu Met Pro Ser Leu Ser Ser
   2085 2090 2095
   His Pro Thr Ser Pro Ala Ala Ala Leu Glu Pro Ala Pro His Pro Gln
   2100 2105 2110
   Gly Ser Gly Ser Val Asn Gly Ser Pro Leu Met Ser Thr Ser Gly Ala
   2115 2120 2125
   Ile Thr Pro Gly Arg Gly Gly Arg Arg Gln Leu Pro Gln Thr Pro Leu
   2130 2135 2140
   Thr Pro Arg Pro Ser Ile Thr Tyr Lys Thr Ala Asn Ser Ser Pro Val
   2145 2150 2155 2160
   His Phe Ala Glu Gly Gln Ser Gly Leu Pro Ala Phe Ser Pro Gly Arg
   2165 2170 2175
   Leu Ser Arg Gly Leu Ser Glu His Asn Ala Leu Leu Gln Lys Glu Pro
   2180 2185 2190
   Leu Ser Gln Pro Leu Ala Pro Gly Ser Arg Ile Gly Ser Asp Pro Tyr
   2195 2200 2205
   Leu Gly Gln Arg Leu Asp Ser Glu Ala Ser Ala His Thr Leu Pro Glu
   2210 2215 2220
   Asp Thr Leu Thr Phe Glu Glu Ala Val Ala Thr Asn Ser Gly Arg Ser
   2225 2230 2235 2240
   Ser Arg Thr Ser Tyr Val Ser Ser Leu Thr Ser Gln Ser His Pro Leu
   2245 2250 2255
   Arg Arg Val Pro Asn Gly Tyr His Cys Thr Leu Gly Leu Asn Thr Gly
   2260 2265 2270
   Val Gly Ala Arg Ala Ser Tyr His His Pro Asp Gln Asp His Trp Cys
   2275 2280 2285
<210> 3
   <211> 27
   <212> PRT
   <213> Conus geographus
<220>
   <221> MOD_RES
   <222> 4, 10, 21
   <223> Xaa=hydroxyproline
<400> 3
   Cys Lys Ser Xaa Gly Ser Ser Cys Ser Xaa Thr Ser Tyr Asn Cys Cys
   1 5 10 15
   Arg Ser Cys Asn Xaa Tyr Thr Lys Arg Cys Tyr
   20 25
<210> 4
   <211> 28
   <212> DNA
   <213> Artificial Sequence
<220>
   <223> primer
<400> 4
   ttgtacagtg agatggaccc tgaggagc 28
<210> 5
   <211> 30
   <212> DNA
   <213> Artificial Sequence
<220>
   <223> primer
<400> 5
   ggcctccagg tcacagtgag gttccttggg 30
<210> 6
   <211> 713
   <212> DNA
   <213> Mus musculus
<400> 6
   ggcattgtac agcgagatgg accctgagga gcgcctgcgt tatgccagca cgcgccatgt    60
   gaggccagac atgaagacac acatggaccg acccctagtg gtggagcctg gtcgagatgg   120
   cttgcgggga cccgttggga gcaagtcaaa gcctgaaggc acggaggcca cagaaagcgc   180
   ggacctacct cgcaggcacc accggcaccg tgatagggac aagacctcag ccacagcacc   240
   tgctggaggc gaacaggaca ggacagaaag caccgagacc ggggcccggg aggaacgtgc   300
   gcgccctcgt cgaagtcaca gcaaggagac tccaggggct gacacgcaag ttgcgctgtg   360
   agcgcagtaa acgtcaccac cggcgcggct ccccggagga ggccactgaa cgggagcttc   420
   ggcgccaccg tgcccaccgg catgcacagg actcaagcaa ggagggcacg gcgccggtgc   480
   ttgtacccaa gggtgagcga cgagcaagac accgaggccc acgcacgggt ccacgtgagg   540
   cagagaacaa cgaggagccc acacgcaggc accgtgcaag gcataaggtg ccacccacac   600
   tgcagccccc agagagggag gctgcagaga aggagagcaa cgcggtggaa ggggataagg   660
   aaacccgaaa tcaccagccc aaggaacctc actgtgacct ggagaccaat gcc          713
<210> 7
   <211> 30
   <212> DNA
   <213> Artificial Sequence
<220>
   <223> primer
<400> 7
   aggcagcctt tgttttagca caaacaaagc 30
<210> 8
   <211> 30
   <212> DNA
   <213> Artificial Sequence
<220>
   <223> primer
<400> 8
   gcctgcttgc cgaatatcat ggtggaaaat 30
<210> 9
   <211> 30
   <212> DNA
   <213> Artificial Sequence
<220>
   <223> primer
<400> 9
   ggtcgagatg gcttgcggga cccgttggga 30
<210> 10
   <211> 30
   <212> DNA
   <213> Artificial Sequence
<220>
   <223> primer
<400> 10
   tggcacctta tgccttgcac ggtgcctgcg 30